# EUROPEAN PATENT APPLICATION

(11) **EP 4 785 990 A1**
(43) Date of publication of application: **05.08.2026**
(21) Application number: 26151922.7
(22) Date of filing: 14.01.2026
(51) Int. Cl.: A61N 1/362, A61N 1/372, A61N 1/375, H04B 13/00

(54) **REDUCING COMMUNICATION BURDEN BETWEEN LEADLESS PACEMAKERS**

(30) Priority: 31.01.2025 US 202563752296 P; 13.01.2026 US 202619448020
(71) Applicant: Pacesetter, Inc., Sylmar, CA 91342 (US)
(72) Inventor: Fishler, Matthew G, Sylmar, CA 91342 (US); Yang, Weiqun, Sylmar, CA 91342 (US)
(74) Representative: Barker Brettell Sweden AB

(57) **Abstract**

Described herein is a multi-chamber implantable LP system (100) comprising at least a first LP (102a, 102b) configured to be implanted in or on a first cardiac chamber, and a second LP (102b, 102a) configured to be implanted in or on a second cardiac chamber. For each cardiac event, of a plurality of cardiac events associated with the first cardiac chamber, the first LP (102a, 102b) is configured to determine for the cardiac event whether at least one of a plurality of predetermined conditions is satisfied. Additionally, the first LP (102a, 102b) is configured to abstain from transmitting a cardiac event message indicative of the cardiac event to the second LP (102b, 102a), or transmit the cardiac event message indicative of the cardiac event to the second LP (102b, 102a), based on whether there is a determination that at least one of the plurality of predetermined conditions is satisfied. Methods for use with such a multi-chamber implantable LP system (100) are also described herein.

## Description

### FIELD OF TECHNOLOGY

Embodiments described herein generally relate to multi-chamber leadless pacemaker systems, leadless pacemakers thereof, and methods for use by multi-chamber leadless pacemaker systems and leadless pacemakers thereof.

### BACKGROUND

An example of a multi-chamber leadless pacemaker (LP) system is a dual-chamber LP system including an atrial leadless pacemaker (aLP) and a ventricular leadless pacemaker (vLP) that utilize implant-to-implant (i2i) communication to coordinate their dual-chamber functionality. More specifically, such a multi-chamber LP system may utilize an i2i communication protocol that requires the aLP to transmit an i2i event message to the vLP whenever the aLP senses an intrinsic atrial event or causes a paced atrial event. Similarly, the i2i communication protocol may also require the vLP to transmit an i2i event message to the aLP whenever the vLP senses an intrinsic ventricular event or causes a paced ventricular event. The i2i event messages sent between the LPs can be conductive communication messages. That is, conductive communication, which is more energy efficient than radio frequency (RF) communication and inductive communication, may be utilized for the i2i communication. Alternatively, RF or inductive communication may be utilized for the i2i communication that takes place between LPs. The longevity of implantable medical devices (IMDs), such as LPs, is dependent on many factors, including the power consumed by such IMDs to perform i2i communication. In certain multi-chamber LP systems, the aLP has a smaller battery than the vLP, and the load impedance for the aLP is lower than that for the vLP.

### SUMMARY

A multi-chamber implantable LP system in accordance with certain embodiments of the present technology comprises a first LP configured to be implanted in or on a first cardiac chamber, and a second LP configured to be implanted in or on a second cardiac chamber. For each cardiac event that is nonconcurrent with the blanking and refractory periods of the first LP, of a plurality of cardiac events associated with the first cardiac chamber that are nonconcurrent with the blanking and refractory periods of the first LP, the first LP is configured to determine for the cardiac event whether at least one of a plurality of predetermined conditions is satisfied. Additionally, the first LP is configured to abstain from transmitting a cardiac event message indicative of the cardiac event to the second LP, or transmit the cardiac event message indicative of the cardiac event to the second LP, based on whether there is a determination that at least one of the plurality of predetermined conditions is satisfied.

In accordance with certain embodiments, for each cardiac event associated with the first cardiac chamber that is nonconcurrent with the blanking and refractory periods of the first LP, of the plurality of cardiac events that are nonconcurrent with the blanking and refractory periods of the first LP, the first LP is configured to: abstain from transmitting the cardiac event message indicative of the cardiac event to the second LP, when there is a determination that none of the plurality of predetermined conditions is satisfied; and transmit the cardiac event message indicative of the cardiac event to the second LP, when there is a determination that at least one of the plurality of predetermined conditions is satisfied.

In accordance with certain embodiments, one of the plurality of predetermined conditions is satisfied when the cardiac event that is nonconcurrent with the blanking and refractory periods of the first LP comprises a paced cardiac event.

In accordance with certain embodiments, the system is configured to operate in one of a tracking mode or a non-tracking mode at a time, and to switch from the tracking mode or the non-tracking mode to the other, and vice versa; and one of the plurality of predetermined conditions is satisfied when the system is operating in the tracking mode and the cardiac event that is nonconcurrent with the blanking and refractory periods of the first LP comprises a sensed cardiac event.

In accordance with certain embodiments, one of the plurality of predetermined conditions is satisfied when a message payload is pending at the first LP, or when a message payload is pending at the first LP that is one of one or more predetermined types of message payload.

In accordance with certain embodiments, a first one of the plurality of predetermined conditions is satisfied when the cardiac event that is nonconcurrent with the blanking and refractory periods of the first LP comprises a paced cardiac event; a second one of the plurality of predetermined conditions is satisfied when the system is operating in the tracking mode and the cardiac event comprises a sensed cardiac event that is nonconcurrent with the blanking and refractory periods of the first LP; and a third one of the plurality of predetermined conditions is satisfied when a message payload is pending at the first LP, or when a message payload is pending at the first LP that is one of one or more predetermined types of message payload.

In accordance with certain embodiments, a first one of the plurality of predetermined conditions is satisfied when the system is operating in the tracking mode and the cardiac event comprises a sensed cardiac event that is nonconcurrent with the blanking and refractory periods of the first LP; and a second one of the plurality of predetermined conditions is satisfied when a message payload is pending at the first LP, or when a message payload is pending at the first LP that is one of one or more predetermined types of message payload.

In accordance with certain embodiments, for each cardiac event associated with the first cardiac chamber that is nonconcurrent with the blanking and refractory periods of the first LP, of the plurality of cardiac events that are nonconcurrent with the blanking and refractory periods of the first LP, the first LP is configured to: abstain from transmitting the cardiac event message indicative of the cardiac event to the second LP, when there is not a determination that at least one of the plurality of predetermined conditions is satisfied; and transmit the cardiac event message indicative of the cardiac event to the second LP, when there is a determination that at least one of the plurality of predetermined conditions is satisfied.

In accordance with certain embodiments, the first LP comprises an aLP configured to be implanted in or on an atrial chamber, and the second LP comprises a vLP configured to be implanted in or on a ventricular chamber. In certain such embodiments, for each atrial event that is nonconcurrent with the blanking and refractory periods of the aLP, of a plurality of atrial events associated with the atrial chamber that are nonconcurrent with the blanking and refractory periods of the aLP, the aLP is configured to: determine for the atrial event whether at least one of a plurality of predetermined conditions is satisfied; and abstain from transmitting an atrial event message indicative of the atrial event to the vLP, or transmit the atrial event message indicative of the atrial event to the vLP, based on whether there is a determination that at least one of the plurality of predetermined conditions is satisfied.

In accordance with certain embodiments, the first LP comprises a vLP configured to be implanted in or on a ventricular chamber, and the second LP comprises an aLP configured to be implanted in or on an atrial chamber. In certain such embodiments, for each ventricular event that is nonconcurrent with the blanking and refractory periods of the vLP, of a plurality of ventricular events associated with the ventricular chamber that are nonconcurrent with the blanking and refractory periods of the vLP, the vLP is configured to: determine for the ventricular event whether at least one of a plurality of predetermined conditions is satisfied; and abstain from transmitting a ventricular event message indicative of the ventricular event to the aLP, or transmit the ventricular event message indicative of the ventricular event to the aLP, based on whether there is a determination that at least one of the plurality of predetermined conditions is satisfied.

In accordance with certain embodiments, each LP, of the first LP and the second LP, comprising a respective controller, one or more respective sense circuits, one or more respective pulse generators, a respective pair of electrodes, and a respective battery that powers the respective controller, the respective one or more sense circuits, and the respective one or more pulse generators of the LP. The respective one or more pulse generators and the respective pair of electrodes, of each LP, are configured to output pacing pulses and output conducted communication pulses. The respective one or more sense circuits and the respective pair of electrodes, of each LP, are configured to sense an electrogram (EGM) and sense conducted communication pulses. In such embodiments, the first LP abstaining from transmitting a said cardiac event message to the second LP reduces an amount of power consumed from the battery of the first LP compared to when the first LP transmits a said cardiac event message to the second LP.

Certain embodiments of the present technology are related to methods for use in a multi-chamber implantable LP system comprising a first LP configured to be implanted in or on a first cardiac chamber and a second LP configured to be implanted in or on a second cardiac chamber. Such a method can be for reducing communication burden and comprises the first LP determining, for each cardiac event that is nonconcurrent with the blanking and refractory periods of the first LP of a plurality of cardiac events associated with the first cardiac chamber that are nonconcurrent with the blanking and refractory periods of the first LP, whether at least one of a plurality of predetermined conditions is satisfied. The method also comprises the first LP selectively abstaining from transmitting a cardiac event message indicative of the cardiac event to the second LP, or transmitting the cardiac event message indicative of the cardiac event to the second LP, based on whether there is a determination for the cardiac event that at least one of the plurality of predetermined conditions is satisfied.

In accordance with certain embodiments, the method includes: the first LP abstaining from transmitting a said cardiac event message indicative of a first said cardiac event to the second LP, in response to the first LP determining for the first cardiac event that none of the plurality of predetermined conditions is satisfied; and the first LP transmitting a said cardiac event message indicative of a second cardiac event to the second LP, in response to the first LP determining for the second cardiac event that at least one of the plurality of predetermined conditions is satisfied.

In accordance with certain embodiments, one of the plurality of predetermined conditions is satisfied when the cardiac event comprises a paced cardiac event.

In accordance with certain embodiments, one of the plurality of predetermined conditions is satisfied when the multi-chamber implantable LP system is operating in the tracking mode and the cardiac event comprises a sensed cardiac event.

In accordance with certain embodiments, one of the plurality of predetermined conditions is satisfied when a message payload is pending at the first LP, or when a message payload is pending at the first LP that is one of one or more predetermined types of message payload.

In accordance with certain embodiments, a first one of the plurality of predetermined conditions is satisfied when the cardiac event comprises a paced cardiac event; a second one of the plurality of predetermined conditions is satisfied when the multi-chamber implantable LP system is operating in the tracking mode and the cardiac event comprises a sensed cardiac event; and a third one of the plurality of predetermined conditions is satisfied when a message payload is pending at the first LP, or when a message payload is pending at the first LP that is one of one or more predetermined types of message payload.

In accordance with certain embodiments, a first one of the plurality of predetermined conditions is satisfied when the multi-chamber implantable LP system is operating in the tracking mode and the cardiac event comprises a sensed cardiac event; and a second one of the plurality of predetermined conditions is satisfied when a message payload is pending at the first LP, or when a message payload is pending at the first LP that is one of one or more predetermined types of message payload.

In accordance with certain embodiments, the method includes: the first LP abstaining from transmitting a said cardiac event message indicative of a first said cardiac event to the second LP, in response to the first LP not determining for the first cardiac event that at least one of the plurality of predetermined conditions is satisfied; and the first LP transmitting a said cardiac event message indicative of a second cardiac event to the second LP, in response to the first LP determining for the second cardiac event that at least one of the plurality of predetermined conditions is satisfied.

In accordance with certain embodiments the first LP comprises an aLP configured to be implanted in or on an atrial chamber, and the second LP comprises a vLP configured to be implanted in or on a ventricular chamber. In certain such embodiments, for each atrial event that is nonconcurrent with the blanking and refractory periods of the aLP of a plurality of atrial events associated with the atrial chamber that are nonconcurrent with the blanking and refractory periods of the first LP, the method comprises: the aLP determining, for the atrial event, whether at least one of a plurality of predetermined conditions is satisfied; and the aLP abstaining from transmitting an atrial event message indicative of the atrial event to the vLP, or transmitting the atrial event message indicative of the atrial event to the vLP, based on whether there is a determination that at least one of the plurality of predetermined conditions is satisfied.

In accordance with other embodiments, the first LP comprises a vLP configured to be implanted in or on a ventricular chamber, and the second LP comprises an aLP configured to be implanted in or on an atrial chamber. In certain such embodiments, for each ventricular event that is nonconcurrent with the blanking and refractory periods of the vLP of a plurality of ventricular events associated with the ventricular chamber that are nonconcurrent with the blanking and refractory periods of the vLP, the method comprises: the vLP determining, for the ventricular event, whether at least one of a plurality of predetermined conditions is satisfied; and the vLP abstaining from transmitting a ventricular event message indicative of the ventricular event to the aLP, or transmitting the ventricular event message indicative of the ventricular event to the aLP, based on whether there is a determination that at least one of the plurality of predetermined conditions is satisfied.

This summary is not intended to be a complete description of the embodiments of the present technology. Other features and advantages of the embodiments of the present technology will appear from the following description in which the preferred embodiments have been set forth in detail, in conjunction with the accompanying drawings and claims.

### BRIEF DESCRIPTION OF THE DRAWINGS

Embodiments of the present technology relating to both structure and method of operation may best be understood by referring to the following description and accompanying drawings, in which similar reference characters denote similar elements throughout the several views:
FIG. 1 illustrates a system formed in accordance with certain embodiments.
FIG. 2 is a block diagram of a single LP in accordance with certain embodiments.
FIG. 3 illustrates an LP in accordance with certain embodiments.
FIG. 4 is a timing diagram demonstrating one embodiment of implant-to-implant (i2i) communication for a paced event.
FIG. 5 is a timing diagram demonstrating one embodiment of i2i communication for a sensed event.
FIG. 6A includes a high level flow diagram used to summarize methods according to certain embodiments of the present technology that are for use with a multi-chamber LP system including at least first and second LPs configured to be implanted respectively in or on first and second cardiac chambers.
FIG. 6B includes a high level flow diagram used to describe additional details of one of the steps introduced in the flow diagram of FIG. 6A.
FIG. 7A includes a high level flow diagram used to summarize methods according to certain embodiments of the present technology that are for use with a multi-chamber LP system including at least an aLP and a vLP.
FIG. 7B includes a high level flow diagram used to describe additional details of one of the steps introduced in the flow diagram of FIG. 7A.

### DETAILED DESCRIPTION

Certain embodiments of the present technology generally relate to multi-chamber LP systems, LPs thereof, and methods for use by multi-chamber LP systems and LPs thereof. Such a multi-chamber LP system, as described in more detail below, can include an aLP implanted in or on an atrial chamber and a vLP implanted in or on a ventricular chamber. As will be described in additional details below, certain embodiments of the present technology enable a first one of the LPs to selectively abstain from transmitting event messages to a second one of the LPs to thereby conserve power used by the first one of the LPs and increase its longevity. For example, certain embodiments enable the aLP to selectively abstain from transmitting atrial event messages to the vLP, to thereby conserve power used by the aLP and increase its longevity. The aLP selectively abstaining from transmitting atrial event messages to the vLP also conserves power of the vLP and thereby the longevity of the vLP is also increased, since the vLP will receive and processes fewer atrial event messages. Other embodiments enable the vLP to selectively abstain from transmitting ventricular event messages to the aLP, to thereby conserve power used by the vLP and increase its longevity. In embodiments where the vLP selectively abstains from transmitting ventricular event messages to the aLP, the aLP also conserves power and thereby the longevity of the aLP is also increased, since the aLP will receive and processes fewer ventricular event message.

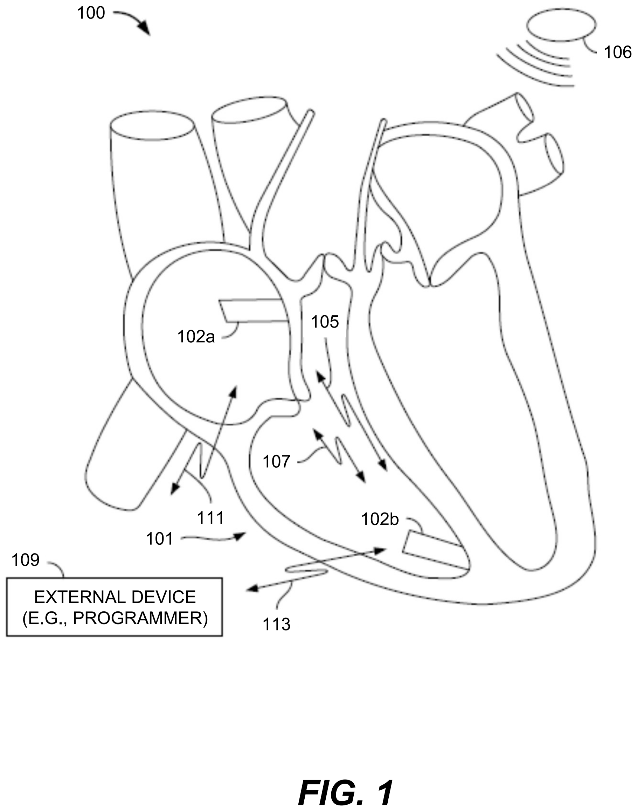

FIGS. 1-5 are used to describe an exemplary multi-chamber leadless pacemaker system with which embodiments of the present technology can be used. A leadless pacemaker can also be referred to herein as a leadless cardiac pacemaker, or more succinctly as an LP. Where a cardiac pacing system includes a non-vascular implantable cardioverter-defibrillator (NV-ICD), such as a subcutaneous-ICD (S-ICD), the NV-ICD may perform certain sensing operations and may communicate with one or more LPs by sending and/or receiving messages to and/or from one or more LPs, as can be appreciated from the below discussion. Where a cardiac pacing system includes a programmer, the programmer may be used to program one or more IMDs (e.g., LPs), download information to one or more IMDs, and/or upload information from one or more IMDs, as can be appreciated from the below description.

FIG. 1 illustrates a system 100 that includes LPs 102a and 102b located in different chambers of a heart 101. The portion of the system 100 that includes the LPs 102 and 102b can be referred to as a multi-chamber LP system 100. The LP 102a is located in the right atrium, while the LP 102b is located in a right ventricle. The LPs 102a and 102b can communicate with one another to inform one another of various local physiologic activities, such as local intrinsic events, local paced events, and/or the like. The LPs 102a and 102b may be constructed in a similar manner, but operate differently based upon which chamber the LP 102a or 102b is located. The LPs 102a and 102b may sometimes be referred to collectively herein as the LPs 102, or individually as an LP 102.

In certain embodiments, the LPs 102a and 102b communicate with one another, and/or with an NV-ICD 106, by conductive communication through the same electrodes that are used for sensing and/or delivery of pacing therapy. The LPs 102a and 102b may also be able to use conductive communication to communicate with an external device 109, e.g., a programmer, having electrodes placed on the skin of a patient within with the LPs 102a and 102b are implanted. It is noted that the term "conductive communication" and the term "conducted communication" are used interchangeably herein. While not shown in FIG. 1 (and not preferred, since it would increase the size and power consumption of the LPs 102a and 102b), the LPs 102a and 102b can potentially include an antenna and/or telemetry coil that would enable them to communicate with one another, the NV-ICD 106 and/or an external device using RF and/or inductive communication. While only two LPs are shown in FIG. 1, it is possible that more than two LPs can be implanted in a patient. For example, to provide for bi-ventricular pacing and/or cardiac resynchronization therapy (CRT), in addition to having LPs implanted in the right atrial (RA) chamber and the right ventricular (RV) chamber, a further LP can be implanted in the left ventricular (LV) chamber. Additionally, or alternatively, a further LP can be implanted in the left atrial (LA) chamber.

In some embodiments, the LPs 102 can be co-implanted with the ICD 106. Each LP 102a uses two or more electrodes located within, on, or within a few centimeters of the housing of the pacemaker, for pacing and sensing at the cardiac chamber, and additionally for bidirectional communication with one another, with the external device 109, and the ICD 106.

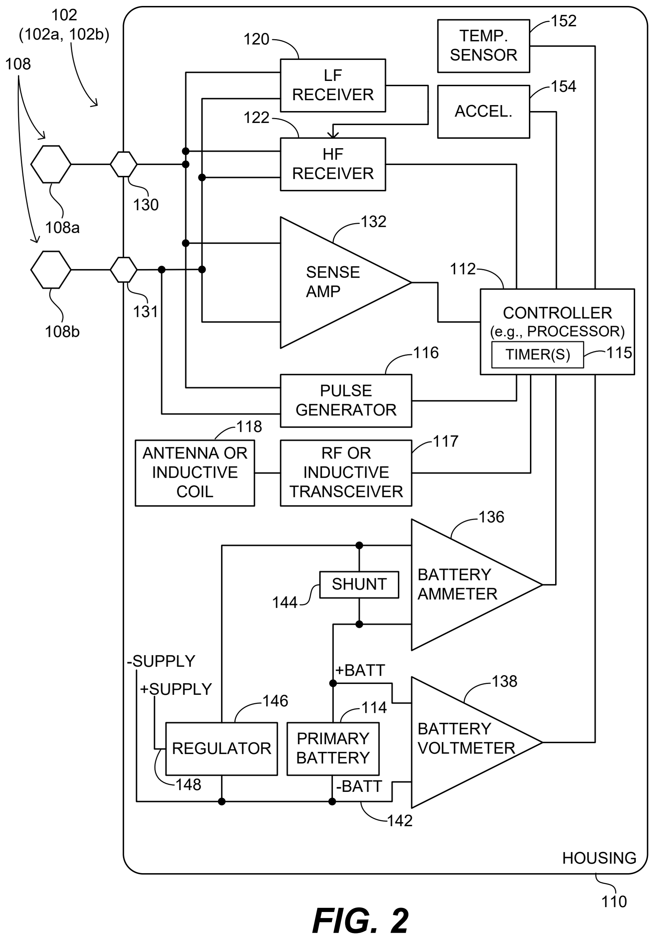

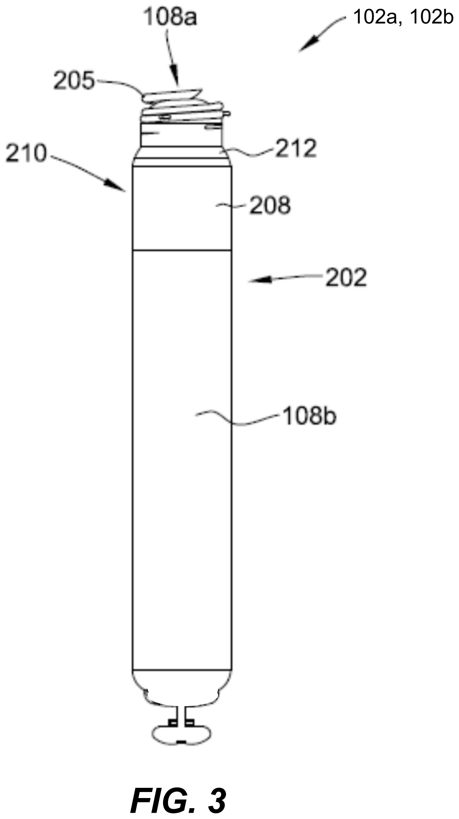

Referring to FIG. 2, a block diagram shows an embodiment for portions of the electronics within LPs 102a, 102b configured to provide conductive communication through the sensing/pacing electrode. One or more of LPs 102a and 102b include at least two leadless electrodes 108a, 108b configured for delivering cardiac pacing pulses, sensing evoked and/or natural cardiac electrical signals, and uni-directional or bi-directional communication. In FIG. 2 (and FIG. 3) the two electrodes shown therein are labeled 108a and 108b. Such electrodes can be referred to collectively as the electrodes 108, or individually as an electrode 108. An LP 102, or other type of IMD, can include more than two electrodes 108, depending upon implementation.

In FIG. 2, each of the LPs 102a, 102b is shown as including first and second receivers 120 and 122 that collectively define separate first and second communication channels 105 and 107 (FIG. 1), (among other things) between LPs 102a and 102b. Although first and second receivers 120 and 122 are depicted, in other embodiments, each LP 102a, 102b may only include a single receiver, or may include additional receivers other than first and second receivers 120 and 122. As will be described in additional detail below, the pulse generator 116 can function as a transmitter that transmits i2i communication signals using the electrodes 108. In certain embodiments, LPs 102a and 102b may communicate over more than just first and second communication channels 105 and 107. In certain embodiments, LPs 102a and 102b may communicate over one common communication channel 105. More specifically, LPs 102a and 102b can communicate conductively over a common physical channel via the same electrodes 108 that are also used to deliver pacing pulses. Usage of the electrodes 108 for communication enables the one or more LPs 102a and 102b to perform antenna-less and telemetry coil-less communication.

The two receivers 120 and 122 shown in FIG. 2 can also be referred to, respectively, as a low frequency (LF) receiver 120 and a high frequency (HF) receiver 122, because the receiver 120 is configured to monitor for one or more signals within a relatively low frequency range (e.g., below 250 kHz) and the receiver 122 is configured to monitor for one or more signals within a relatively high frequency range (e.g., above 250 kHz). In certain embodiments, the receiver 120 (and more specifically, at least a portion thereof) is always enabled and monitoring for a wakeup notice, which can simply be a wakeup pulse, within a specific low frequency range (e.g., between 1 kHz and 250 kHz); and the receiver 122 is selectively enabled by the receiver 120. The receiver 120 is configured to consume less power than the receiver 122 when both the first and second receivers are enabled. Accordingly, the receiver 120 can also be referred to as a low-power receiver 120, and the receiver 122 can also be referred to as a high-power receiver 122. The low-power receiver 120 is incapable of receiving signals within the relatively high frequency range (e.g., above 250 kHz), but consumes significantly less power than the high-power receiver 122. This way the low-power receiver 120 is capable of always monitoring for a wakeup notice without significantly depleting the battery (e.g., 114) of the LP. In accordance with certain embodiments, the high-power receiver 122 is selectively enabled by the low-power receiver 120, in response to the low-power receiver 120 receiving a wakeup notice, so that the high-power receiver 122 can receive the higher frequency signals, and thereby handle higher data throughput needed for effective i2i communication without unnecessarily and rapidly depleting the battery of the LP (which the high-power receiver 122 may do if it were always enabled).

In accordance with certain embodiments, when one of the LPs 102a and 102b senses an intrinsic event or delivers (or is about to deliver) a paced event, the corresponding LP 102a, 102b can transmit an event message to the other LP 102a, 102b. Where an event message originates from an LP (e.g., 102a) implanted in or on an atrial chamber (e.g., the right atrial chamber), the event message can be referred to more specifically as an atrial event message. Where an event message originates from an LP (e.g., LP 102b) implanted in or on a ventricular chamber (e.g., the right ventricular chamber), the event message can be referred to more specifically as a ventricular event message.

For example, when an atrial LP 102a senses or paces an atrial event, the atrial LP 102a may transmit an atrial event message including an event marker indicative of a nature of the event (e.g., intrinsic/sensed atrial event, paced atrial event). When a ventricular LP 102b senses or paces a ventricular event, the ventricular LP 102b may transmit a ventricular event message including an event marker indicative of a nature of the event (e.g., intrinsic/sensed ventricular event, paced ventricular event). In certain embodiments, each LP 102a, 102b can transmit a paced event message to the other LP 102a, 102b preceding delivery of an actual pace pulse so that the remote LP can blank its sense inputs in anticipation of that remote pace pulse (to prevent inappropriate crosstalk sensing by the remote LP). In alternative embodiments, each LP 102a, 102b may abstain from transmitting a paced event message to the other LP 102a, 102b following delivery of a pace pulse, and the remote LP relies on an alternative technique for avoiding crosstalk sensing.

Where an event message is transmitted by a first LP to a second LP to inform the second LP of an intrinsic event sensed by the first LP, the event message can be referred to more specifically as a sensed event message. Where the first LP is an aLP (e.g., 102a), the sensed event message can be referred to more specifically as an atrial sensed event message. Where the first LP is a vLP (e.g., 102a), the sensed event message can be referred to more specifically as a ventricular sensed event message. Where an event message is transmitted by a first LP to a second LP to inform the second LP of a paced event caused (or about to be caused) by the first LP, the event message can be referred to more specifically as a paced event message. Where the first LP is an aLP (e.g., 102a), the paced event message can be referred to more specifically as an atrial paced event message. Where the first LP is a vLP (e.g., 102a), the paced event message can be referred to more specifically as a ventricular paced event message.

The implant event messages, which are also referred to herein as cardiac event messages or event messages, may be formatted in various manners. As one example, each event message may include a leading trigger pulse (also referred to as a notice trigger pulse, wakeup notice, wakeup pulse or wakeup signal) followed by an event marker. The leading trigger pulse is transmitted over a first channel (e.g., with a pulse duration of approximately 4 µs to approximately 1.0 msec and/or within a fundamental frequency range of approximately 1 kHz to approximately 250 kHz). The leading trigger pulse indicates that an event marker is about to be transmitted over a second channel (e.g., within a higher frequency range). The event marker can then be transmitted over the second channel.

The event markers may include data indicative of one or more events (e.g., a sensed intrinsic atrial activation for an atrial located LP, a sensed intrinsic ventricular activation for a ventricular located LP, a paced atrial event for delivery of a pacing pulse by an atrial located LP, a paced ventricular event for delivery of a pacing pulse by a ventricular located LP). The event markers may include different markers for intrinsic and paced events. The event markers may also indicate start or end times for timers (e.g., an AV interval, a blanking interval, etc.). Optionally, the implant event message may include a message segment that includes additional/secondary information.

Optionally, the LP (or other IMD) that receives any i2i communication signal from another LP (or other IMD) or from an external device may transmit a receive acknowledgement indicating that the receiving LP (or other IMD) received the i2i communication signal. In certain embodiments, where an LP or another type of IMD expects to receive an i2i communication signal within a window, and fails to receive the i2i communication signal within the window, the LP or other type of IMD may transmit a failure-to-receive acknowledgement indicating that the receiving LP or other type of IMD failed to receive the i2i communication signal. Other variations are also possible and within the scope of the embodiments described herein.

The event messages enable the LPs 102a, 102b to deliver synchronized therapy and additional supportive features (e.g., measurements, etc.). To maintain coordinated therapy, each of the LPs 102a and 102b can be made aware (through the event messages) when an event occurs in the chamber containing the other LP 102a, 102b. Some embodiments described herein provide efficient and reliable processes to maintain synchronization between LPs 102a and 102b without maintaining continuous communication between LPs 102a and 102b. In accordance with certain embodiments herein, low-power event messages/signaling may be maintained between LPs 102a and 102b synchronously or asynchronously.

For synchronous event signaling, LPs 102a and 102b may maintain synchronization and regularly communicate at a specific interval. Synchronous event signaling allows the transmitter (e.g., pulse generator 116) and receivers in each LP 102a, 102b to use limited (or minimal) power as each LP 102a, 102b is only powered for a small fraction of the time in connection with transmission and reception. For example, LP 102a, 102b may transmit/receive (Tx/Rx) communication messages in time slots having duration of 10-20 µs, where the Tx/Rx time slots occur periodically (e.g., every 10-20 msec).

In accordance with certain embodiments herein, LPs 102a and 102b may utilize multi-stage receivers that implement a staged receiver wakeup scheme in order to improve reliability yet remain power efficient. Each of LPs 102a and 102b may include first and second receivers 120 and 122 that operate with different first and second activation protocols and different first and second receive channels. For example, first receiver 120 may be assigned a first activation protocol that is "always on" (also referred to as always awake) and that listens over a first receive channel that has a lower fundamental frequency range/pulse duration as compared to the fundamental frequency range assigned to the second receive channel.

In accordance with certain embodiments, the first receiver 120 may maintain the first channel active (awake) at all times (including when the second channel is inactive (asleep)) in order to listen for messages from a remote LP. The second receiver 122 may be assigned a second activation protocol that is a triggered protocol, in which the second receiver 122 becomes active (awake) in response to detection of trigger events over the first receive channel (e.g., when the incoming signal corresponds to the LP wakeup notice, activating the second channel at the local LP). The terms active, awake and enabled are used interchangeably herein.

Still referring to FIG. 2, each LP 102a, 102b is shown as including a controller 112 and a pulse generator 116. The controller 112 can include, e.g., a microprocessor (or equivalent control circuitry), RAM and/or ROM memory, logic and timing circuitry, state machine circuitry, and I/O circuitry, but is not limited thereto. The controller 112 can further include, e.g., timing control circuitry to control the timing of the stimulation pulses (e.g., pacing rate, atrioventricular (AV) delay, atrial interconduction (A-A) delay, or ventricular interconduction (V-V) delay, etc.). For example, the controller 112 of the LP 102a, 102b can be used to implement one or more timers 115, including but not limited to, an AV interval timer and a ventricular-to-ventricular interval (VV interval) timer. The controller 112 of the LP 102a can similarly be used to implement one or more timers 115 that may be used by the LP 102a, 102b. Such timing control circuitry may also be used for the timing of refractory periods, blanking intervals (also referred to herein as blanking periods), noise detection windows, evoked response windows, alert intervals, marker channel timing, and so on.

The controller 112 can further include other dedicated circuitry and/or firmware/software components that assist in monitoring various conditions of the patient's heart and managing pacing therapies. The controller 112 and the pulse generator 116 may be configured to transmit event messages, via the electrodes 108a, 108b, in a manner that does not inadvertently capture the heart in the chamber where LP 102a, 102b is located, such as when the associated chamber is not in a refractory state. In addition, a LP 102a, 102b that receives an event message may enter an "event refractory" state (or event blanking state) following receipt of the event message. The event refractory/blanking state may be set to extend for a determined period of time after receipt of an event message in order to avoid the receiving LP 102a, 102b from inadvertently sensing another signal as an event message that might otherwise cause retriggering. For example, the receiving LP 102a, 102b may detect a measurement pulse from another LP 102a, 102b or an external device 109, such a programmer, as shown in FIG. 1.

In accordance with certain embodiments herein, an external device 109, such as a programmer, may communicate over a programmer-to-LP channel 113, with an LP 102a, 102b utilizing the same communication scheme. The external device 109, such as programmer, may listen to the event message transmitted between LP 102a, 102b and synchronize programmer to implant communication such that the external device 109, such as programmer, does not transmit communication signals until after an implant to implant messaging sequence is completed.

While not shown, a communication capacitor can be provided in the LP 102a, 102b. The communication capacitor may be used to transmit event signals having higher voltage for the event message pulses to improve communication, such as when the LPs 102a and 102b experience difficulty sensing event messages. The high voltage event signaling may be used for implants with high signal attenuation or in the case of a retry for an ARQ (automatic repeat request) handshaking scheme.

In some embodiments, the individual LP 102a, 102b can comprise a hermetic housing 110 configured for placement on or attachment to the inside or outside of a cardiac chamber and at least two leadless electrodes 108a, 108b proximal to the housing 110 and configured for bidirectional communication with at least one other device 106 within or outside the body.

FIG. 2 depicts a single LP 102a, 102b and shows the LP's functional elements substantially enclosed in a hermetic housing 110. The LP 102a, 102b has at least two electrodes 108a, 108b located within, on, or near the housing 110, for delivering pacing pulses to and sensing electrical activity from the muscle of the cardiac chamber, and for bidirectional communication with at least one other device within or outside the body. Hermetic feedthroughs 130, 131 conduct electrode signals through the housing 110. The housing 110 contains a primary battery 114 to supply power for pacing, sensing, and communication. The housing 110 also contains sense circuits 132 for sensing cardiac activity from the electrodes 108a, 108b, receivers 120, 122 for receiving information from at least one other device via the electrodes 108a, 108b, and the pulse generator 116 for generating pacing pulses for delivery via the electrodes 108a, 108b and also for transmitting information to at least one other device via the electrodes 108a, 108b. In an embodiment, the housing 110 can optionally contain circuits for monitoring device health, for example a battery current monitor 136 and a battery voltage monitor 138, and can contain circuits for controlling operations in a predetermined manner.

The electrodes 108a, 108b can be configured to communicate bidirectionally among the multiple LPs and/or the implanted NV-ICD 106 to coordinate pacing pulse delivery and optionally other therapeutic or diagnostic features using messages that identify an event at an individual pacemaker originating the message and a pacemaker receiving the message react as directed by the message depending on the origin of the message. An LP 102a, 102b that receives the event message reacts as directed by the event message depending on the message origin or location. In some embodiments or conditions, the two or more leadless electrodes 108a, 108b can be configured to communicate bidirectionally among the one or more LPs 102a and/or the NV-ICD 106 and transmit data including designated codes for events detected or created by an individual pacemaker. Individual pacemakers can be configured to issue a unique code corresponding to an event type and a location of the sending pacemaker.

Moreover, information communicated on the incoming channel can also include an event message from another LP signifying that the other LP has sensed a heartbeat or has delivered a pacing pulse, and identifies the location of the other pacemaker. For example, LP 102b may receive and relay an event message from LP 102a to the external device 109, such as a programmer. Similarly, information communicated on the outgoing channel can also include a message to another LP or pacemakers, or to the NV-ICD 106, that the sending LP has sensed a heartbeat or has delivered a pacing pulse at the location of the sending pacemaker.

Referring again to FIGS. 1 and 2, the system 100 may comprise an NV-ICD 106, such as in the form of an ICD, in addition to one or more LPs 102a, 102b configured for implantation in electrical contact with a cardiac chamber and for performing cardiac rhythm management functions in combination with the implantable NV-ICD 106. The implantable NV-ICD 106 and the LPs 102a, 102b can be configured to perform i2i communication via conduction through body tissue and/or wireless transmission between transmitters and receivers in accordance with the embodiments discussed herein. Each of the LPs comprises at least two leadless electrodes 108a, 108b configured for delivering cardiac pacing pulses, sensing evoked and/or natural cardiac electrical signals, and optionally transmitting information to the co-implanted NV-ICD 106.

Each of the LPs 102a, 102b can be configured for operation in a particular location and a particular functionality at manufacture and/or at programming by an external programmer. Bidirectional communication among the multiple LPs can be arranged to communicate notification of a sensed heartbeat or delivered pacing pulse event and encoding type and location of the event to another implanted pacemaker or pacemakers. The LP 102a, 102b receiving the communication decodes the information and responds depending on location of the receiving LP and predetermined system functionality.

In some embodiments, the LPs 102a and 102b are configured to be implantable in any chamber of the heart, namely either atrium (RA, LA) or either ventricle (RV, LV). Furthermore, for dual-chamber configurations, multiple LPs may be co-implanted (e.g., one in the RA and one in the RV, one in the RV and one in the coronary sinus proximate the LV). Certain pacemaker parameters and functions depend on (or assume) knowledge of the chamber in which the pacemaker is implanted (and thus with which the LP is interacting; e.g., pacing and/or sensing). Some non-limiting examples include sensing sensitivity, an evoked response algorithm, use of AF suppression in a local chamber, blanking & refractory periods, etc. Accordingly, each LP needs to know an identity of the chamber in which the LP is implanted, and processes may be implemented to automatically identify a local chamber associated with each LP.

Also shown in FIG. 2, the primary battery 114 has positive terminal 140 and negative terminal 142. Current from the positive terminal 140 of primary battery 114 flows through an optional shunt 144 to an optional regulator circuit 146 to create a positive voltage supply 148 suitable for powering the remaining circuitry of the LP 102. The optional shunt 144 enables the optional battery current monitor 136 to provide the controller (e.g., processor) 112 with an indication of battery current drain and indirectly of device health. The illustrative power supply can be a primary battery 114.

Referring to FIG. 2, the LP is shown as including an optional temperature sensor 152. The optional temperature sensor can be any one of various types of well-known temperature sensors, or can be a future developed temperature sensor. The optional temperature sensor 152 can be used in various manners. For example, the optional temperature sensor 152 can be used to detect an activity level of the patient to adjust a pacing rate, i.e., for use in rate responsive pacing. When a person starts to exercise their core body temperature initially dips, and then after exercising for a prolonged period of time the person's core body temperature will eventually rise. Thereafter, when the person stops exercising their core body temperature will return to its baseline. Accordingly, the controller 112 can be configured to detect an activity level of a patient based on core blood temperature measurements obtained using the optional temperature sensor 152.

Referring to FIG. 2, the LP is also shown as including an optional accelerometer 154 which can be hermetically contained within the housing 110. The optional accelerometer 154 can be any one of various types of well-known accelerometers, or can be a future developed accelerometer. For one example, the optional accelerometer 154 can be or include, e.g., a MEMS (micro-electromechanical system) multi-axis accelerometer of the type exploiting capacitive or optical cantilever beam techniques, or a piezoelectric accelerometer that employs the piezoelectric effect of certain materials to measure dynamic changes in mechanical variables. For example, the optional accelerometer 154 can be used to detect an activity level of the patient to adjust a pacing rate, i.e., for use in rate responsive pacing. It would also be possible to use outputs of both the optional accelerometer 154 and the optional temperature sensor 152 to monitor the activity level of a patient. Alternatively, or additionally, a patient's activity level can be monitored based on their heart rate, as detected from an electrogram (EGM) sensed using the electrodes 108a, 108b, and/or sensed using a plethysmography signal obtained using an optional plethysmography sensor (not shown) or an optional heart sound sensor (not shown), but not limited thereto.

The controller 112 of the LP 102a can detect intrinsic atrial events from an EGM that is sensed by the LP 102a. Alternatively, or additionally, the controller 112 of the LP 102a can detect intrinsic atrial events from one or more signals sensed by the optional accelerometer 154 thereof and/or from a heart sounds signal sensed by an optional microphone (not shown) of the LP 102a, 102b. The controller 112 of the LP 102b can detect intrinsic ventricle events from an EGM that is sensed by the LP 102b. Alternatively, or additionally, the controller 112 of the LP 102b can detect intrinsic ventricular events from one or more signals sensed by the optional accelerometer 154 thereof and/or from a heart sounds signal sensed by an optional microphone (not shown) of the LP 102b. In certain embodiments, each of the LPs 102a, 102b includes only one of the optional temperature sensor 152 and the optional accelerometer 154.

In various embodiments, LP 102a, 102b can manage power consumption to draw limited power from the battery, thereby reducing device volume. Each circuit in the LP 102a, 102b can be designed to avoid large peak currents. For example, cardiac pacing can be achieved by discharging a tank capacitor (not shown) across the pacing electrodes. Recharging of the tank capacitor is typically controlled by a charge pump circuit. In a particular embodiment, the charge pump circuit is throttled to recharge the tank capacitor at constant power from the battery.

In some embodiments, the controller 112 of an LP 102a, 102b can access signals on the electrodes 108a, 108b and can examine output pulse duration from another pacemaker for usage as a signature for determining triggering information validity and, for a signature arriving within predetermined limits, activating delivery of a pacing pulse following a predetermined delay of zero or more milliseconds. The predetermined delay can be preset at manufacture, programmed via an external programmer, or determined by adaptive monitoring to facilitate recognition of the triggering signal and discriminating the triggering signal from noise. In some embodiments or in some conditions, the controller 112 can examine an output pulse waveform from another LP for usage as a signature for determining triggering information validity and, for a signature arriving within predetermined limits, activating delivery of a pacing pulse following a predetermined delay of zero or more milliseconds.

Instead of or in addition to the LP 102a, 102b utilizing conductive communication to communicate with another LP, another type of NV-ICD 106, and/or an external device 109, the LP 102a, 102b can, in an embodiment, include an RF or inductive transceiver 117 that is coupled to the controller 112, and the RF or inductive transceiver 117 can be coupled to an antenna or inductive coil 118, to thereby enable the LP 102a, 102b to utilize RF communication and/or inductive communication to communicate with another LP, another type of NV-ICD 106, and/or an external device 109. In other words, the communication performed by the LP 102a, 102b can be conductive communication, RF communication, or inductive communication, or any combination thereof. In an embodiment, the LP 102a, 102b does not include any RF transceiver 117 or any antenna 118. In another embodiment, the LP 102a, 102b does not include any inductive transceiver 117 or any inductive coil 118. In a further embodiment, the LP 102a, 102b does not include any RF or inductive transceiver 117 or any antenna or inductive coil 118.

FIG. 3 shows an LP 102a, 102b. The LP can include a hermetic housing 202 with electrodes 108a, 108b disposed thereon. As shown, electrode 108a can be separated from but surrounded partially by a fixation mechanism 205, and the electrode 108b can be disposed on the housing 202. The fixation mechanism 205 can be a fixation helix, a plurality of hooks, barbs, or other attaching features configured to attach the pacemaker to tissue, such as heart tissue. The electrodes 108a, 108b are examples of the electrodes 108 shown in and discussed above with reference to FIG. 2.

The housing 202 can also include an electronics compartment 210 within the housing that contains the electronic components necessary for operation of the LP 102a, 102b, including, e.g., a pulse generator, receiver, a battery, and a controller for operation. The hermetic housing 202 can be adapted to be implanted on or in a human heart, and can be cylindrically shaped, rectangular, spherical, or any other appropriate shapes, for example.

The housing 202 can comprise a conductive, biocompatible, inert, and anodically safe material such as titanium, 316L stainless steel, or other similar materials. The housing 202 can further comprise an insulator disposed on the conductive material to separate electrodes 108a, 108b. The insulator can be an insulative coating on a portion of the housing 202 between the electrodes 108a, 108b, and can comprise materials such as silicone, polyurethane, parylene, or another biocompatible electrical insulator commonly used for implantable medical devices. In the embodiment of FIG. 3, a single insulator 208 is disposed along the portion of the housing 202 between electrodes 108a, 108b. In some embodiments, the housing 202 itself can comprise an insulator instead of a conductor, such as an alumina ceramic or other similar materials, and the electrodes 108a, 108b can be disposed upon the housing 202.

As shown in FIG. 3, the LP 102a, 102b can further include a header assembly 212 to isolate the electrodes 108a, 108b. The header assembly 212 can be made from PEEK, tecothane or another biocompatible plastic, and can contain a ceramic to metal feedthrough, a glass to metal feedthrough, or other appropriate feedthrough insulator as known in the art.

The electrodes 108a, 108b can comprise pace/sense electrodes, or return electrodes. A low-polarization coating can be applied to the electrodes, such as sintered platinum, platinum-iridium, iridium, iridium-oxide, titanium-nitride, carbon, or other materials commonly used to reduce polarization effects, for example. In FIG. 3, electrode 108a can be a pace/sense electrode and electrode 108b can be a return electrode. The electrode 108b can be a portion of the conductive housing 202 that does not include an insulator 208.

Several techniques and structures can be used for attaching the housing 202 to the interior or exterior wall of the heart. A helical fixation mechanism 205, can enable insertion of the device endocardially or epicardially through a guiding catheter. A torqueable catheter can be used to rotate the housing and force the fixation device into heart tissue, thus affixing the fixation device 205 (and also the electrode 108a in FIG. 3) into contact with stimulable tissue. Electrode 108b can serve as an indifferent electrode for sensing and pacing. The fixation mechanism 205 may be coated partially or in full for electrical insulation, and a steroid-eluting matrix may be included on or near the device to minimize fibrotic reaction, as is known in conventional pacing electrode-leads.

### Implant-to-Implant Event Messaging

LPs 102a, 102b can utilize implant-to-implant (i2i) communication through event messages to coordinate operation with one another in various manners. The terms i2i communication, i2i event messages, and i2i event markers are used interchangeably herein to refer to event related messages and IMD/IMD operation related messages transmitted from an implanted device and directed to another implanted device (although external devices, e.g., a programmer, may also receive i2i event messages). In certain embodiments, LP 102a and LP 102b operate as two independent leadless pacers maintaining beat-to-beat dual-chamber functionality via a "Master/Slave" operational configuration. For descriptive purposes, the ventricular LP 102b shall be referred to as "vLP" and the atrial LP 102a shall be referred to as "aLP". The LP 102 that is designated as the master device (e.g. vLP) may implement all or most dual-chamber diagnostic and therapy determination algorithms. For purposes of the following illustration, it is assumed that the vLP is a "master" device, while the aLP is a "slave" device. Alternatively, the aLP may be designated as the master device, while the vLP may be designated as the slave device. The master device orchestrates most or all decision-making and timing determinations (including, for example, rate-response changes).

In accordance with certain embodiments, methods are provided for coordinating operation between first and second LPs configured to be implanted in (or on) first and second chambers of the heart. In such a method, the first LP transmits an event marker through conductive communication through electrodes located along a housing of the first LP, the event marker indicative of one of a local paced or sensed event. The method also comprises the second LP detects, over a sensing channel, the event marker. The method further comprises the second LP identifies the event marker based on a predetermined pattern configured to indicate that an event of interest has occurred in a remote chamber. In response to the identifying operation, the second LP initiates a related action.

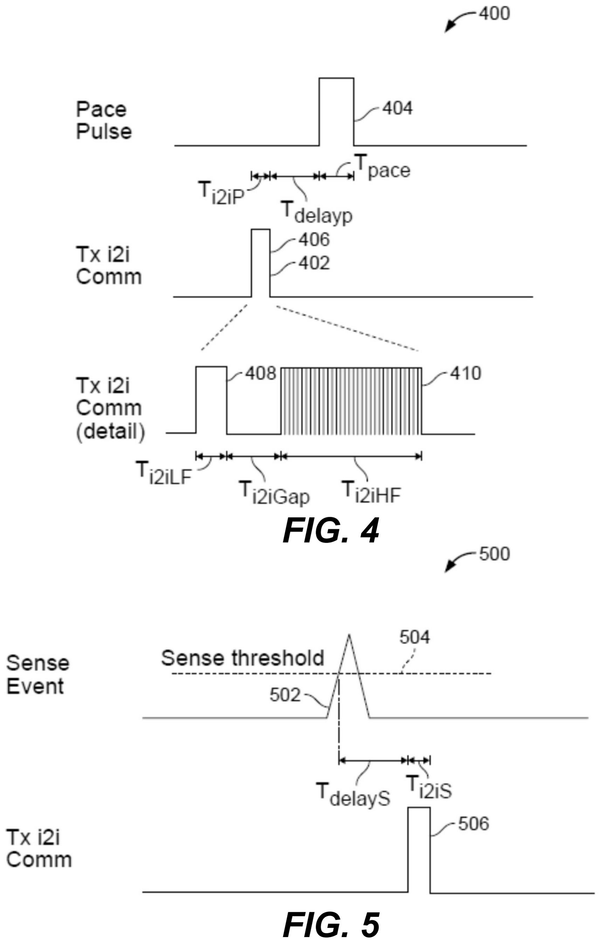

FIG. 4 is a timing diagram 400 demonstrating one example of an i2i communication for a paced event. The i2i communication may be transmitted, for example, from LP 102a to LP 102b. As shown in FIG. 4, in this embodiment, an i2i transmission 402 is sent prior to delivery of a pace pulse 404 by the transmitting LP (e.g., LP 102). This enables the receiving LP (e.g., LP 102b) to prepare for the remote delivery of the pace pulse. The i2i transmission 402 includes an envelope 406 that may include one or more individual pulses. For example, in this embodiment, envelope 406 includes a low frequency pulse 408 followed by a high frequency pulse train 410. Low frequency pulse 408 lasts for a period T_{i2iLF}, and high frequency pulse train 410 lasts for a period T_{i2iHF}. The end of low frequency pulse 408 and the beginning of high frequency pulse train 410 are separated by a gap period, T_{i2iGap}.

As shown in FIG. 4, the i2i transmission 402 lasts for a period Ti2iP, and pace pulse 404 lasts for a period Tpace. The end of i2i transmission 402 and the beginning of pace pulse 404 are separated by a delay period, TdelayP. The delay period may be, for example, between approximately 0.0 and 10.0 milliseconds (msec), particularly between approximately 0.1 msec and 2.0 msec, and more particularly approximately 1.0 msec. The term approximately, as used herein, means +/- 10% of a specified value.

FIG. 5 is a timing diagram 500 demonstrating one example of an i2i communication for a sensed event. The i2i communication may be transmitted, for example, from LP 102a to LP 102b. As shown in FIG. 5, in this embodiment, the transmitting LP (e.g., LP 102a detects the sensed event when a sensed intrinsic activation 502 crosses a sense threshold 504. A predetermined delay period, T_{delayS}, after the detection, the transmitting LP transmits an i2i transmission 506 that lasts a predetermined period T_{i2iS}. The delay period may be, for example, between approximately 0.0 and 10.0 milliseconds (msec), particularly between approximately 0.1 msec and 2.0 msec, and more particularly approximately 1.0 msec.

As with i2i transmission 402, i2i transmission 506 may include an envelope that may include one or more individual pulses. For example, similar to envelope 406, the envelope of i2i transmission 506 may include a low frequency pulse followed by a high frequency pulse train.

Optionally, wherein the first LP is located in an atrium and the second LP is located in a ventricle, the first LP produces an AS or AP event marker to indicate that an atrial sensed (AS) event has occurred or an atrial paced (AP) event has occurred or will occur in the immediate future. For example, the AS and AP event markers may be transmitted following the corresponding AS or AP event. Alternatively, the first LP may transmit the AP event marker slightly prior to delivering an atrial pacing pulse. In certain embodiments, wherein the first LP is located in an atrium and the second LP is located in a ventricle, the second LP initiates an atrioventricular (AV) interval in response to receiving an AS or AP event marker from the first LP. It is also possible that the second LP can initiate an AV interval in response to receiving an AP event marker from the first LP, and the second LP can initiate a PV interval in response to receiving an AS event marker from the first LP, where the duration of AV interval may be greater than the duration of the PV interval by about 25 msec, but not limited thereto. Additionally, the second LP can initiate a post atrial ventricular blanking (PAVB) interval after receiving an AP event marker from the first LP.

In accordance with some embodiments, communication and synchronization between the aLP and vLP is implemented via conducted communication of markers/commands in the event messages (per i2i communication protocol). As explained above, conducted communication represents event messages transmitted from the sensing/pacing electrodes at frequencies outside the RF or Wi-Fi frequency range. Alternatively, the event messages may be conveyed over communication channels operating in the RF or Wi-Fi frequency range. The figures and corresponding description below illustrate non-limiting examples of markers that may be transmitted in event messages. The figures and corresponding description below also include the description of the markers and examples of results that occur in the LP that receives the event message. Table 1 represents exemplary event markers sent from the aLP to the vLP, while Table 2 represents exemplary event markers sent from the vLP to the aLP. In the master/slave configuration, AS event markers are sent from the aLP each time that an atrial event is sensed outside of the post ventricular atrial blanking (PVAB) interval or some other alternatively-defined atrial blanking period. The AP event markers are sent from the aLP each time that the aLP delivers a pacing pulse in the atrium. The aLP may restrict transmission of AS markers, whereby the aLP transmits AS event markers when atrial events are sensed both outside of the PVAB interval and outside the post ventricular atrial refractory period (PVARP) or some other alternatively-defined atrial refractory period. Alternatively, the aLP may not restrict transmission of AS event markers based on the PVARP, but instead transmit the AS event marker every time an atrial event is sensed.

**Table 1**

| "A2V" Markers / Commands (*i.e.,* from aLP to vLP) | | |
|---|---|---|
| *Marker* | *Description* | *Result in vLP* |
| AS | Notification of a sensed event in atrium (if not in PVAB or PVARP) | • Initiate PV interval (if not in PVAB or PVARP) |
| AP | Notification of a paced event in atrium | • Initiate PAVB |
| | | • Initiate AV interval (if not in PVAB or PVARP) |

As shown in Table 1, when an aLP transmits an event message that includes an atrial sensed (AS) event marker (indicating that the aLP sensed an intrinsic atrial event), the vLP initiates a PV interval timer (also known as an AS-VP interval timer). If the aLP transmits an AS event marker for all sensed events, then the vLP would preferably first determine that a PVAB or PVARP interval is not active before initiating the PV interval timer. If however the aLP transmits an AS event marker only when an intrinsic signal is sensed outside of a PVAB or PVARP interval, then the vLP could initiate the PV interval timer upon receiving an AS event marker without first checking the PVAB or PVARP status. When the aLP transmits an atrial paced (AP) event marker (indicating that the aLP delivered or is about to deliver a pace pulse to the atrium), the vLP initiates a PAVB timer and an AV interval timer (also known as an AP-VP interval timer), provided that a PVAB or PVARP interval is not active. The vLP may also blank its sense amplifiers to prevent possible crosstalk sensing of the remote pace pulse delivered by the aLP.

**Table 2**

| "V2A" Markers / Commands (*i.e.,* from vLP to aLP) | | |
|---|---|---|
| *Marker* | *Description* | *Result in aLP* |
| VS | Notification of a sensed event in ventricle | • Initiate PVAB |
| | | • Initiate PVARP |
| VP | Notification of a paced event in ventricle | • Initiate PVAB |
| | | • Initiate PVARP |
| AP | Command to deliver immediate pace pulse in atrium | • Deliver immediate pace pulse to atrium |

As shown in Table 2, when the vLP senses a ventricular event, the vLP transmits an event message including a ventricular sensed (VS) event marker, in response to which the aLP may initiate a PVARP interval timer. When the vLP delivers or is about to deliver a pace pulse in the ventricle, the vLP transmits ventricular paced (VP) event marker. When the aLP receives the VP event marker, the aLP initiates the PVAB interval timer and also the PVARP interval timer. The aLP may also blank its sense amplifiers to prevent possible crosstalk sensing of the remote pace pulse delivered by the vLP. The vLP may also transmit an event message containing an AP command marker to command the aLP to deliver an immediate pacing pulse in the atrium upon receipt of the command without delay.

The foregoing event markers are examples of a subset of markers that may be used to enable the aLP and vLP to maintain full dual-chamber functionality. In one embodiment, the vLP may perform all dual-chamber algorithms, while the aLP may perform atrial-based hardware-related functions, such as PVAB, implemented locally within the aLP. In this embodiment, the aLP is effectively treated as a remote 'wireless' atrial pace/sense electrode. In another embodiment, the vLP may perform most but not all dual-chamber algorithms, while the aLP may perform a subset of diagnostic and therapeutic algorithms. In an alternative embodiment, vLP and aLP may equally perform diagnostic and therapeutic algorithms. In certain embodiments, decision responsibilities may be partitioned separately to one of the aLP or vLP. In other embodiments, decision responsibilities may involve joint inputs and responsibilities.

In an embodiment, ventricular-based pace and sense functionalities are not dependent on any i2i communication, in order to provide safer therapy. For example, in the event that LP to LP (i2i) communication is lost (prolonged or transient), the multi-chamber implantable LP system 100 may automatically revert to safe ventricular-based pace/sense functionalities as the vLP 102bis running all of the necessary algorithms to independently achieve these functionalities. For example, the vLP 102b may revert to a VVI mode as the vLP 102b does not depend on i2i communication to perform ventricular pace/sense activities. Once i2i communication is restored, the multi-chamber implantable LP system 100 can automatically resume dual-chamber functionalities.

Messages that are transmitted between LPs 102 (e.g., the aLP 102a and the vLP 102b) can be referred to herein generally as i2i messages, since they are implant-to-implant messages. As noted above, such messages can include event markers that enable one LP to inform the other LP of a paced event or a sensed event. For example, in certain embodiments, whenever the aLP 102a senses an atrial event or paces the right atrium, the aLP 102a may transmit an i2i message to the vLP 102b to inform the vLP 102b of the sensed or paced event in the atrium. In response to receiving such an i2i message, the vLP 102b may start one or more timers that enable the vLP 102b to sense or pace in the right ventricle. Similarly, the vLP 102b may transmit an i2i message to the aLP 102a whenever the vLP 102b senses a ventricular event or paces the right ventricle.

The i2i messages that are sent between LPs 102 may be relatively short messages that simply allow a first LP to inform a second LP of an event that was sensed by the first LP or caused (paced) by the first LP, and vice versa. Such i2i messages can be referred to herein as event marker i2i messages, or more succinctly as event i2i messages, or even more succinctly as event messages. The i2i messages that are sent between LPs 102, in certain instances, can be extended i2i messages that include (in addition to an event marker) an extension. In certain embodiments, an extended i2i message includes an event marker (e.g., 9 bits), followed by an extension indicator (e.g., 2 bits), followed by an extended message payload portion (e.g., 17 bits), followed by a cyclic redundancy check (CRC) code (e.g., 6 bits) or some other type of error detection and correction code. In other words, some i2i messages can include a message payload in addition to an event marker.

In certain embodiments, whenever an i2i message is sent by an LP (or other type of IMD, such as a S-ICD), the i2i message will include an extension indicator so that the receiving LP knows whether or not the i2i message it receives includes an extension portion. In such embodiments, even a relatively short event i2i message will include an extension indicator. The extension indicator (e.g., 2 bits) is used by the LP (or other IMD) sending the i2i message to indicate, to the LP receiving the i2i message, whether or not the i2i message is an extended i2i message. In certain embodiments, if the LP receiving an i2i message determines based on the extension indicator bits that the received i2i message is not an extended i2i message, then the LP receiving the i2i message can ignore any bits that follow the extension bits. In such a case, the LP receiving the i2i message only decodes the event marker. On the other hand, if the LP receiving an i2i message determines based on the extension indicator bits that the received i2i message is an extended i2i message, then the LP receiving the i2i message will also decode the bits that follow the extension bits, and determine based on a CRC code (or other type of error detection and correction code), whether or not the i2i message is a valid message. If the extended i2i message is a valid i2i message, then the LP receiving the extended i2i message will as appropriate modify its operation, update one or more parameters, and/or the like, based on information included in the extended i2i message. In certain embodiments, event i2i messages that are not extended i2i messages do not include any error detection and correction code.

In an extended i2i message, the event marker bits and the extension indicator bits are located, respectively, in an event marker field and an extension indicator field of an i2i message packet. In certain embodiments, the extended portion (that follows the event marker bits and the extension indicator bits) includes message bits (in a message field) and rate indicator bits (in a rate indicator field), which are parts of the message payload. The message payload can alternatively, or additionally, include other types of fields, such as an acknowledgement field that is used in certain situations for one LP to acknowledge reception of an i2i message from another LP of certain (e.g., critical) types of message.

More generally, various types of information may be included within the message payload of an extended i2i message. For example, the message payload can include a pacing rate indicator that enables one LP to inform another LP of a pacing rate. Assume that a dual-chamber LP system 100 provides rate responsive pacing, wherein a pacing rate is adjusted in dependence on a patient's physical activity as detected, e.g., using an accelerometer 154, temperature sensor 152, and/or other type of sensor of an LP 102. In such a dual-chamber LP system 100, the vLP 102b may inform the aLP 102a of the rate at which the patient's heart should be paced so that the aLP 102a and vLP 102b can perform synchronized pacing. To achieve this, the vLP 102b can send a pacing rate indicator to the aLP 102a in the message payload of an i2i message. The pacing rate indicator can, e.g., be a value indicating a pacing rate value (e.g., 80 bpm), a code that the aLP 102a that can look up (e.g., in a stored look up table) and corresponds to a pacing rate value, or a value that the aLP 102a feeds into an equation to determine the pacing rate, but is not limited thereto. Alternatively, the pacing rate indicator can be beat-to-beat interval value (e.g., 0.75 seconds), a code that the aLP 102a can look up and corresponds to a beat-to-beat interval value, or a value that the aLP 102a feeds into an equation to determine the beat-to-beat interval, but is not limited thereto. Other variations are also possible and within the scope of the embodiments described herein. The message payload can alternatively or additionally include, for example, a recommended replacement time (RRT) indicator, an automatic mode switch (AMS) entry indicator, an AMS exit indicator, a magnet entry indicator, or a magnet exit indicator. For still another example, the message payload of an i2i message transmitted from an aLP 102a to a vLP 102b can include atrial interval information, such as an average atrial rate interval or a Filtered Atrial Rate Interval (FARI), based upon which the vLP can set a rate responsive refractory period duration.

As noted above, in the Background, the longevity of IMDs, such as LPs, is dependent on many factors, including the power consumed by such IMDs to perform i2i communication. Multi-chamber LP systems 100 may utilize i2i communication to provide for coordinated cardiac pacing. One possible i2i communication protocol that can be utilized by a dual-chamber LP system requires that an i2i event message is transmitted from one LP to another LP upon each paced and sensed event associated with the LP. For example, such an i2i communication protocol may require that an aLP 102a transmits an atrial event message to the vLP 102b each time the aLP 102a detects an atrial sensed event and each time the aLP 102a causes (or is about to cause) an atrial paced event. An atrial event message that informs the vLP 102b of an atrial sensed event can be referred to herein more specifically as an atrial sensed event message. An atrial event message that informs the vLP 102b of an atrial paced event can be referred to herein more specifically as an atrial paced event message. Accordingly, the general use of the term atrial event message, as used herein, can refer to either an atrial sensed event message or an atrial paced event message. Where conductive communication is used to transmit such i2i messages, i.e., where i2i messages are conductive communication messages, the communication between LPs is relatively power efficient. Nevertheless, any opportunity to reduce the communication output burden of an LP will translate into a corresponding increase in the LP's overall longevity. Further, where an aLP 102a has a smaller battery than a vLP 102b, as may be the case in some dual-chamber LP systems 100, it is even more important to reduce the communication output burden of the aLP 102a. This is also the case because an output impedance for an aLP 102a may be lower than that for a vLP 102b.

For patients indicated for a dual-chamber pacemaker system 100 due to AV Node block, their atrial-based sinus node generally functions well wherein it generates an appropriate intrinsic rhythm, but the subsequent propagation of that intrinsic signal from the atria to the ventricles through the AV node is blocked or substantially delayed. As such, a dual-chamber LP system 100 functions to bridge the dysfunctional AV node by sensing the intrinsic atrial events and then correspondingly pacing the ventricle after an appropriate atrioventricular (AV) delay. Such an AV delay can also be referred to herein as an AV interval. Where a dual-chamber LP system 100 is implanted in such a patient, an aLP 102a senses each intrinsic atrial event and may transmit an atrial event message to the vLP 102b to notify (aka inform) the vLP 102b of each atrial sensed event. Upon receipt of that atrial event message, the vLP 102b may initiate an AV interval timer or a PV interval timer and then pace the ventricle when that timer expires. The vLP 102b may also transmit a ventricular event message back to the aLP 102a notifying the aLP 102a of the ventricular paced event, or when appropriate, of a ventricular sensed event. In such a dual-chamber LP system 100, the aLP 102a may also transmit an atrial event message to the vLP 102b each time the aLP 102a causes (or is about to cause) a paced atrial event, and the vLP 102b may respond accordingly to receiving such a message. Such a response by the vLP 102b can be to initiate (start) an AV interval timer, which can also be referred to as an AV delay timer. After the AV interval timer is initiated (started), the vLP 102b can pace the ventricular chamber (in or one which the vLP 102b is implanted), i.e., can cause a paced ventricular event, in response to the AV interval timer expiring (timing out) without an intrinsic ventricular event being detected. The AV interval timer is reset in response to the paced atrial event, or in response to a sensed atrial event, depending upon which occurs first during a cardiac cycle. The AV interval timer can be a count-up timer or a count-down timer, depending upon the specific implementation. Similarly, each of the other timers described herein can be a count up or count down timer. Each such timer 115 can be implemented by a controller (e.g., a processor) 112 of an LP 102 and/or by timing circuitry of the LP.

Certain embodiments of the present technology described herein provide for an updated i2i communication protocol between LPs 102 of a multi-chamber LP system 100, wherein the updated i2i communication protocol conserves energy in order to increase device longevity of at least one of the LPs by being selective when cardiac event messages are transmitted from one LP to another. In other words, rather than transmitting a cardiac event message from one LP to another LP every single cardiac cycle, an LP will selectively transmit, and selectively abstain from transmitting, cardiac event messages to another LP. The multi-chamber LP system 100 with which embodiments of the present technology are used can be a dual-chamber LP system that includes LPs configured to pace and/or sense in two cardiac chambers, an example of which was described above with reference to FIG. 1. It is also possible that the multi-chamber LP system 100, with which embodiments of the present technology can be used, includes three or four LPs, configured to pace and/or sense in three or four cardiac chambers. For much of the following discussion, the multi-chamber LP system 100 will be described as including a first LP configured to be implanted in or on a first cardiac chamber, and a second LP configured to be implanted in or on a second cardiac chamber. The multi-chamber LP system 100 can optionally also include a third LP configured to be implanted in or on a third cardiac chamber. Additionally, the multi-chamber LP system 100 can optionally include a fourth LP configured to be implanted in or on a fourth cardiac chamber. The first cardiac chamber can be, e.g., the right atrial cardiac chamber (aka the right atrium) and the second cardiac chamber can be, e.g., the right ventricular cardiac chamber (aka the right ventricle), or vice versa. The third chamber can be, e.g., the left atrial cardiac chamber (aka the left atrium) and the fourth cardiac chamber can be, e.g., the left ventricular cardiac chamber (aka the left ventricle), or vice versa. If the multi-chamber LP system 100 includes only two LPs, then the multi-chamber LP system 100 can be referred to more specifically as a dual-chamber LP system. However, it should be understood that the term multi-chamber LP system 100 as used herein means that the LP system 100 includes two or more LPs each of which is configured to be implanted in or on a different respective cardiac chamber. Accordingly, it should be understood that a dual-chamber LP system is a type of multi-chamber LP system 100. In certain embodiments, the multi-chamber LP system 100 includes a first LP 102a configured to be implanted in or on a first cardiac chamber, and a second LP 102b configured to be implanted in or on a second cardiac chamber, where the first cardiac chamber is the right atrial chamber (aka the right atrium) and the second cardiac chamber is the right ventricular chamber (aka the right ventricle), or vice versa.

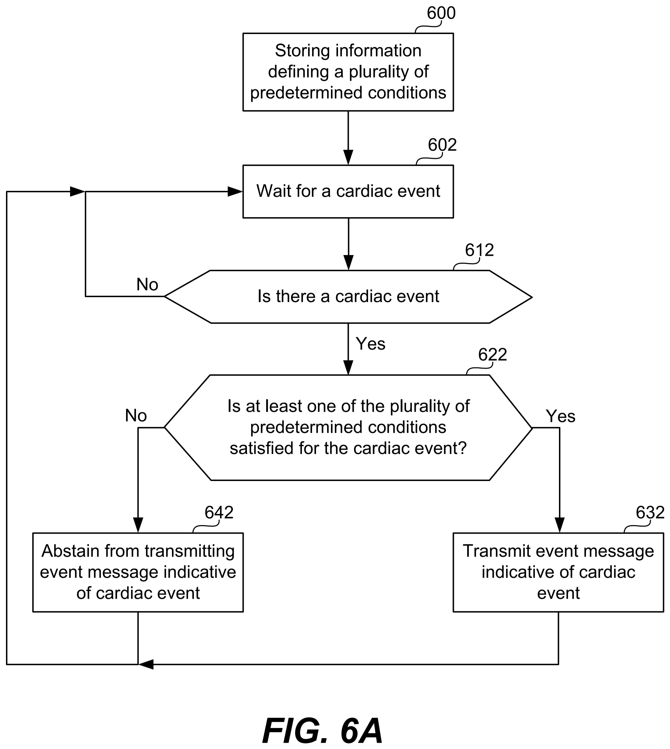

The high level flow diagram of FIG. 6A will now be used to summarize methods according to certain embodiments of the present technology that are for use with a multi-chamber LP system including at least first and second LPs, wherein the first LP is configured to be implanted in or on a first cardiac chamber, and the second LP is configured to be implanted in or on a second cardiac chamber. Referring to FIG. 6A, the steps performed therein are performed by the first LP that is configured to selectively communicate with the second LP of the multi-chamber LP system, which can be a dual-chamber LP system, but is not limited thereto. Step 600 involves the first LP storing information defining a plurality of predetermined conditions. Such information can be stored in memory (not shown) of or communicatively coupled to a controller (e.g., 112) of the first LP. The predetermined conditions can be specified by default by the manufacturer of the first LP, or can be programmable and thereby selectable by a user (e.g., a physician or clinician). Step 602 involves waiting for a cardiac event associated with the chamber in or on which the first LP is implanted that is nonconcurrent with the blanking and refractory periods of the first LP. If the first LP is an aLP, then step 602 involves the aLP waiting for an atrial event that is nonconcurrent with the blanking and refractory periods of the aLP. Alternatively, if the first LP is a vLP, then step 602 involves the vLP waiting for a ventricular event that is nonconcurrent with the blanking and refractory periods of the vLP. The event that is waited for at step 602 can be an intrinsic event that is sensed by the first LP that is nonconcurrent with the blanking and refractory periods of the first LP, which event can also be referred to as a sensed event. The event that is waited for at step 602 can alternatively be a paced event that is caused by the first LP, or is about to be caused by the first LP. In other words, the cardiac event referred to at step 602 and at other steps of FIGS. 6A (and 6B, 7A and 7B) can be a sensed event corresponding to an intrinsic cardiac activity sensed by the first LP at the first cardiac chamber or a paced event corresponding to application of a pacing pulse by the first LP at the first cardiac chamber.

Step 612 involves determining whether there is a cardiac event associated with the chamber in or on which the first LP is implanted, which as noted above can be an intrinsic event or a paced event. Accordingly, the answer to the determination at step 612 will be Yes in response to the first LP sensing an intrinsic event. Where the first LP is configured to deliver pacing stimulation to a cardiac chamber (in or on which the first LP is implanted) in response to an interval timer expiring without an intrinsic event being detected (during an interval that is reset each time a cardiac event is sensed or paced), then the answer to the determination at step 612 will be Yes in response to the interval timer expiring. More generally, the answer to the determination at step 612 can be Yes just prior to (e.g., approximately 1 to 5 msec before) the first LP delivers pacing stimulation to the cardiac chamber (in or on which the first LP is implanted), while the first LP is delivering the pacing stimulation to the cardiac chamber, or just after (e.g., approximately 1 to 5 msec after) the first LP delivered the pacing stimulation to the cardiac chamber.

When the answer to the determination at step 612 is No, flow returns to step 602 and the first LP continues to wait for a cardiac event (associated with the chamber in or on which the first LP is implanted), which cardiac event as noted above can be a sensed event or a paced event that is nonconcurrent with the blanking and refractory periods of the first LP. When the answer to the determination at step 612 is Yes, either because an intrinsic event is sensed, or a paced event is caused (or about to be caused), then flow goes to step 622. More generally, flow can go from step 612 to step 622 in response to a sensed event, a paced cardiac event having been caused, or a paced cardiac event about to be caused.

At step 622 there is a determination of whether at least one of the plurality of predetermined conditions is satisfied for the cardiac event (which had caused the answer to the determination at step 612 to be Yes). The predetermined conditions, as noted above, can be specified by default by the manufacturer of the first LP, or can be programmable and thereby selectable by a user (e.g., a physician or clinician).

When the answer to the determination at step 622 is Yes, flow goes to step 632. Step 632 involves transmitting, from the first LP to the second LP (e.g., from an aLP to a vLP, or from a vLP to an aLP), an event message indicative of the cardiac event (which had caused the answer to the determination at step 612 to be Yes). Following step 632, flow returns to step 602 at which the first LP waits for a next cardiac event associated with the chamber in or on which the first LP is implanted that is nonconcurrent with the blanking and refractory periods of the first LP.

When the answer to the determination at step 622 is No, flow goes to step 642. Step 642 involves the first LP abstaining from transmitting to the second LP an event message indicative of the cardiac event (which had caused the answer to the determination at step 612 to be No). Following step 642, flow returns to step 602 at which the first LP waits for a next cardiac event associated with the chamber in or on which the first LP is implanted that is nonconcurrent with the blanking and refractory periods of the first LP. The flow diagram of FIG. 6A could have equivalently shown flow returning from step 622 directly back to step 602 when the answer to the determination at step 622 is No, since in such a flow diagram the transmitting at step 632 would be skipped (and thus, abstained from being performed) whenever that answer to the determination at step 622 is No.

Embodiments described herein can be used by a multi-chamber LP system (e.g., a dual-chamber LP system) to conserve substantially more energy (and thereby further increase device longevity) than would be the case if only a single type of predetermined condition being satisfied (e.g., the multi-chamber LP system being in a non-tracking mode) caused a first LP of the system to abstain from transmitting a cardiac event message to a second LP of the system. Further, by not just using a single type of predetermined condition as the deciding factor for determining whether or not a cardiac event message is to be transmitted from the first LP to the second LP, there can be situations where a cardiac event message is transmitted from the first LP to the second LP when the system is in a non-tracking mode because it is beneficial to the operation of the multi-chamber LP system. In other words, for reasons other than enabling the second LP to track the first LP it may be beneficial for the first LP to still send a cardiac event message to the second LP when the system is in a non-tracking mode, e.g., to allow for blanking by the second LP, and/or to enable the first LP to transfer a useful payload message to the second LP as soon as possible.

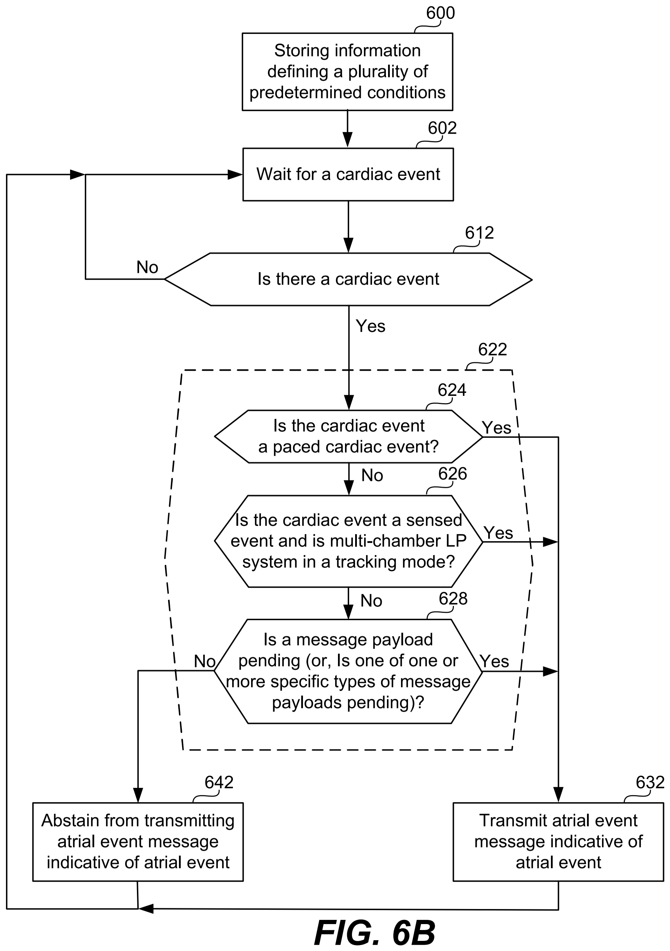

Additional details of step 622, in accordance to specific embodiments of the present technology, will now be described with reference to FIG. 6B. Referring to FIG. 6B, steps 602 and 612 are the same as steps 602 and 612 in FIG. 6A, and thus, need not be described again. In FIG. 6B, steps 624, 626, and 628 are sub-steps of step 622 introduced above with reference to FIG. 6A. As explained above, flow goes to step 622 when the answer to the determination at 612 is Yes. More specifically, when there is a sensed cardiac event or a paced cardiac event (which had caused the answer to the determination at step 612 to be Yes), flow goes to step 624.

At step 624 there is a determination of whether the cardiac event (which had caused the answer to the determination at step 612 to be Yes) is a paced cardiac event. If the answer to the determination at step 624 is Yes, then flow goes to step 632, at which the first LP transmits to the second LP a cardiac event message indicative of the paced cardiac event, which had caused the answer to the determination at step 624 to be Yes. Such an event message indicative of the paced cardiac event can also be referred to more succinctly as a paced event message. It is beneficial for the first LP to transmit a paced event message to the second LP because receipt thereof by the second LP can cause the second LP to initiate a pacing blanking response that reduces the probability of the second LP falsely detecting a paced event (caused by the first LP) as a sensed event of the second cardiac chamber (in or on which the second LP is implanted). More specifically, in certain embodiments, receipt of the paced event message by the second LP causes the second LP to initiate a blanking interval (also referred to herein as a blanking period) during which the second LP blanks (ignores or disables) sense inputs of second LP to prevent the second LP from falsely detecting a pacing pulse (delivered to the first cardiac chamber) as a depolarization of the second cardiac chamber. If the answer to the determination at step 624 is No, then flow goes to step 626.

At step 626 there is a determination of whether the cardiac event is a sensed event that is nonconcurrent with the blanking and refractory periods of the first LP and the multi-chamber LP system (e.g., dual-chamber LP system) is in a tracking mode, as opposed to a non-tracking mode. If the answer to the determination is at step 626 is Yes, i.e., if the cardiac event is a sensed event that is nonconcurrent with the blanking and refractory periods of the first LP and the multi-chamber LP system is indeed in a tracking mode, then flow goes to step 632, at which the first LP transmits to the second LP a cardiac event message. When the multi-chamber LP system is in a tracking mode, the second LP delivers pacing stimulation to a second cardiac chamber (in or on which the second LP is implanted) at a specified delay following a sensed or paced event of the first cardiac chamber, if the second LP does not detect an intrinsic cardiac event during a specified interval. Example tracking modes that the first and second LPs of the multi-chamber LP system can collectively provide include DDD, DDDR, VDD, VDDR, DDT, and DDTR operational modes. Accordingly, when the multi-chamber LP system is in a tracking mode, the second LP will attempt to pace the second cardiac chamber (in or on which the second LP is implanted) at the same rate as the cardiac event rate of the first cardiac chamber (in or on which the first LP is implanted) and offset by a specified delay. Accordingly, it is beneficial for the first LP to transmit cardiac event messages to the second LP when the multi-chamber LP system is in a tracking mode to maintain coordinated contractions of the first and second cardiac chambers.

By contrast, when the multi-chamber LP is in a non-tracking mode, the second LP delivers pacing stimulation to the second cardiac chamber (in or on which the second LP is implanted) at a rate that is independent of the sensed cardiac event rate of the first cardiac chamber (in or on which the first LP is implanted). Example non-tracking modes that the first and second LPs of the multi-chamber LP system can collectively provide include DDI, DDIR, VDI, VDIR, DOO, and DOOR operational modes. The multi-chamber LP system may switch from a tracking mode to a non-tracking mode when the cardiac event rate of the first cardiac chamber (in or one which the first LP is implanted) exceeds a specified tracking rate threshold. The switch from a tracking mode to a non-tracking mode beneficially prevents the second LP from pacing the second chamber (in or on which the second LP is implanted) at a dangerously high rate. Because the second LP delivers pacing stimulation to the second cardiac chamber (in or on which the second LP is implanted) at a rate that is independent of the sensed cardiac event rate of the first cardiac chamber (in or on which the first LP is implanted), while the multi-chamber LP system is in a non-tracking mode, there may be little or no benefit of the second LP receiving cardiac event messages from the first LP on a beat-by-beat basis (i.e., every cardiac cycle) while the multi-chamber LP system is in a non-tracking mode. Accordingly, the first LP can conserve energy by not transmitting cardiac event messages to the second LP while the multi-chamber LP system is in a non-tracking mode, since those cardiac event messages may not be used by the second LP to control the timing of the pacing performed by the second LP. If the answer to the determination at step 626 is No, then flow goes to step 628. The cardiac event rate of an atrial chamber can also be referred to herein as an atrial event rate, or more succinctly as an atrial rate. The cardiac event rate of a ventricular chamber can also be referred to herein as a ventricular event rate, or more succinctly as a ventricular rate.

At step 628 there is a determination of whether a message payload is pending that is to be transmitted as part of the cardiac event message indicative of the cardiac event that is nonconcurrent with the blanking and refractory periods of the first LP (which had caused the answer to the determination at step 612 to be Yes). If the answer to the determination at step 628 is Yes, i.e., if a message payload is indeed pending, then flow goes to step 632. At step 632 the first LP transmits to the second LP a cardiac event message that includes the message payload that is pending. It is also noted that if a message payload is pending when a cardiac event message is transmitted in response to a determination at step 624 or step 626 being Yes, then such a pending message payload will also be sent in those instances. For example, if the cardiac event is a paced event and there is also a message payload pending, when the cardiac event message indicative of the paced cardiac event is transmitted from the first LP to the second LP (in response to the answer to the determination at step 624 being Yes) the message payload that is pending will also be sent as part of that cardiac event message. For another example, if the multi-chamber LP system is in a tracking mode when there is a sensed (or paced) cardiac event of the first cardiac chamber, if a message payload is pending at that time, when the cardiac event message indicative of the sensed (or paced) cardiac event is transmitted from the first LP to the second LP the message payload that is pending will also be sent as part of that cardiac event message.

A benefit of transmitting a cardiac event message including a message payload when the message payload is pending is that information (e.g., one or more indicators and/or commands) included in the message payload can be used by the second LP to modify its operation as soon as possible. For example, if a pending message payload includes a pacing rate indicator, then the first LP transmitting the pacing rate indicator in the message payload of a cardiac event message enables the second LP (that receives the cardiac event message) to adjust its pacing rate responsive to the pacing rate indicator included in the message payload. For another example, if a pending message payload includes an automatic mode switch (AMS) entry indicator, then transmitting the AMS entry indicator in the message payload of a cardiac event message enables the second LP to switch from a tracking mode to a non-tracking mode. For still another example, if a pending message payload includes an AMS exit indicator, then transmitting the AMS exit indicator in the message payload of a cardiac event message enables the second LP (that receives the cardiac event message) to switch from a non-tracking mode to a tracking mode. Auto Mode Switching (AMS) is a standard dual-chamber pacemaker feature that, upon detection of a high atrial rate (e.g., during atrial fibrillation or flutter), provides automatic transition from an AV synchronous pacing mode to one without atrial tracking so as to avoid non-physiologically high ventricular rates that might otherwise result in adverse/symptomatic hemodynamic cardiac performance. Conversely, when the high atrial rate reverts to a more physiologic rate, AMS functionality will terminate and the pacemaker system will again transition back to an AV synchronous pacing mode. For a specific example, an aLP may want to send a cardiac event message that includes an AMS entry indicator within its message payload to the vLP whenever the aLP enters an AMS mode, and the aLP may want to send a cardiac event message that includes an AMS exit indicator within its message payload to the vLP whenever the aLP exits the AMS mode. Other types of indicators that may be included within a message payload, that may be beneficial to transmit from the first LP to the second LP as soon as possible, include a magnet entry indicator, and a magnet exit indicator, but are not limited thereto. A first LP may want to send a magnet entry indicator to a second LP when the first LP senses a magnetic field that is used to cause the multi-chamber LP system to operate in a magnet mode. The specific functionality of a magnet mode for an LP may vary depending upon how the LP is programmed, but in general, one or more operations of the LP will be different when the LP is in its magnet mode compared to when the LP is not in its magnet mode.

The determinations that are made at the steps 624, 626, and 628 in FIG. 6B, which can be considered sub-steps of the step 622 introduced in the discussion of FIG. 6A, are collectively an example of the determination performed at step 622. In other words, sub-steps 624, 626, and 628 can be performed to determine whether at least one of a plurality of predetermined conditions is satisfied at step 622. Examples of the predetermined conditions (which when at least one of them is satisfied results in a cardiac event message indicative of a cardiac event being transmitted from the first LP to the second LP at an instance of step 632) include: a cardiac event is a paced cardiac event; the cardiac event is a sensed event and the multi-chamber LP system is in a tracking mode; and a message payload is pending. It is also possible that step 622 includes just two of the sub-steps 624, 626, and 628 (e.g., just sub-steps 624 and 626, just sub-steps 624 and 628, or just sub-steps 626, and 628). It is also within the scope of the embodiments described herein that there can be additional and/or alternative predetermined conditions, where if at least one of the predetermined conditions is found to be satisfied, results in a cardiac event message indicative of a cardiac event being transmitted from the first LP to the second LP. In other words, there can be additional and/or alternative determinations performed at step 622, or at sub-steps thereof.

Further, it is noted that the order of the steps 624, 626, and 628 can be rearranged, wherein as noted above such steps can be referred to as sub-steps of step 622. More specifically, the order of the steps can instead be steps 626, 628, 624, in that order. Alternatively, the order of the steps can instead be steps 628, 624, 626, in that order. In still another alternative, the order of the steps can be step 628, 626, 624, in that order. When the order of such steps are rearranged, flow will go to step 632 whenever the answer to any one of the determinations in such steps is Yes. Additionally, when the order of such steps are rearranged, flow should go step 642 when the answer to all of the determinations in such steps is No. In other words, whichever one of the steps 624, 626, and 628 is the last to be performed should include an arrow to step 642 (or equivalent back to step 602) responsive to the answer to the determination made at the last performed step being No.

Referring again to step 628, in certain embodiments a cardiac event message will be sent from the first LP to the second LP if a message payload is pending, regardless of the type of message payload that is pending. In other words, in certain embodiments the answer to the determination at step 628 will be Yes, regardless of the type of message payload that is pending.

In alternative embodiments, not all message payloads are treated the same. Rather, in such alternative embodiments only one or more specific types of message payloads if pending will result in the answer to the determination at step 628 being Yes. More specially, in such an embodiment, at step 628 there is a determination of whether a message payload, which is to be transmitted from the aLP to the vLP as part of the atrial event message indicative of the atrial event that is nonconcurrent with the blanking and refractory periods of the aLP, is pending and is one of one or more predetermined types of message payload. The one or more predetermined types of message payload can be those that are deemed to be sufficiently important such that they should be provided from the first LP to the second LP as soon as possible, so that the second LP can modify its behavior accordingly as soon as possible. In such embodiments, for other types of message payload which are deemed to be less important, the operation of the multi-chamber LP system may be unaffected if the first LP does not provide the message payload to the second LP as soon as possible, e.g., if the message payload simply includes a housekeeping type of command, or some other type of command or indicator for which it is not important that the second LP modify its behavior accordingly as soon as possible. Examples of one or more predetermined types of message payload that can be deemed to be sufficient important such that they should be provided from the first LP to the second LP as soon as possible, and thus should result in the answer to step 628 being Yes, are message payloads that include: a command to switch from a tracking mode to a non-tracking mode (e.g., an AMS entry command), a command to switch from a non-tracking mode to a tracking mode (e.g., an AMS exit command), a magnet entry indicator, or a magnet exit indicator, but are not limited thereto.

As noted above, a reason it is beneficial for the first LP to transmit a paced cardiac event message to the second LP is because receipt thereof by the second LP causes the second LP to initiate a blanking interval during which the second LP blanks sense inputs of second LP to prevent the second LP from falsely detecting a pacing pulse delivered to the first cardiac chamber as a depolarization of the second cardiac chamber. However, in embodiments where the second LP is configured to detect crosstalk caused by the first LP delivering pacing stimulation, the second LP can initiate a blanking interval in response to the second LP detecting such crosstalk, instead of in response to the second LP receiving a paced cardiac event message from the first LP. In other words, in embodiments where the second LP is configured to detect crosstalk caused by the first LP delivering pacing stimulation to the first cardiac chamber (in or on which the first LP is implanted), the second LP is not reliant on paced cardiac event messages to initiate a blanking interval, but rather, the second LP can instead initiate a blanking interval in response to detecting the crosstalk caused by the first LP delivering the pacing stimulation to the first cardiac chamber. Accordingly, when the multi-chamber LP system is in a non-tracking mode, the second LP can rely on crosstalk detection to initiate a blanking interval.

The second LP can be configured to detect crosstalk, for example, using one of the techniques described in the description of FIGS. 2-8 of U.S. Patent No. 10,182,765, titled "Systems and Methods for Classifying Signals of Interest in a Cardiac Rhythm Management Device". For example, in order for the second LP to detect crosstalk, which occurs due to the first LP delivering pacing stimulation (to the cardiac chamber in or on which the first LP is implanted), the second LP can also include a separate crosstalk sense circuit that has a passband that is shifted to a significantly higher frequency than the passband of the intrinsic activity sense amplifier (e.g., 132), to enable the crosstalk sense circuit to be more discriminative of the relatively fast rising and falling edges of crosstalk that may be caused by a pacing pulse produced by the first LP. Such a crosstalk sense circuit can be implemented, for example, by the LF receiver 120, or by another sense circuit not specifically shown in FIG. 2. Using such sense circuits, the second LP, and more specifically a controller (e.g., 112) thereof, can classify a sensed signal being evaluated as being crosstalk where the sensed signal is passed by the crosstalk sense circuit. This is just one example of circuits and techniques that the second LP can use to perform possible crosstalk monitoring. A lower cutoff frequency of the crosstalk sensing circuit can be, e.g., greater than or equal to 500 Hz. More generally, crosstalk can be detected if a sensed signal has energy above a threshold within a specified frequency range in which crosstalk, when present, is expected to be. The use of other circuits and/or techniques to monitor for and detect crosstalk can alternatively be used.

In an embodiment, where the second LP is configured to detect crosstalk caused by the first LP delivering pacing stimulation to the first cardiac chamber (in or on which the first LP is implanted), and the second LP is configured to start a blanking interval in response to detecting such crosstalk, step 624 can be removed from the flow diagram of FIG. 6B. In such an embodiment, when the answer to the determination at step 612 is Yes, flow can go directly from step 612 to step 626. Alternatively, when the answer to the determination at step 612 is Yes, flow can go directly from step 612 to step 628, and the order of steps 626 and 628 can be rearranged such that step 628 is performed prior to step 626.

In embodiments summarized above with reference to FIGS. 6A and 6B, for each cardiac event associated with the first cardiac chamber in or on which first LP is implanted that is nonconcurrent with the blanking and refractory periods of the first LP, of a plurality of cardiac events that are nonconcurrent with the blanking and refractory periods of the first LP, the first LP is configured to: abstain from transmitting the cardiac event message indicative of the cardiac event to the second LP, when there is a determination that none of the plurality of predetermined conditions is satisfied; and transmit the cardiac event message indicative of the cardiac event to the second LP, when there is a determination that at least one of the plurality of predetermined conditions is satisfied. In alternative yet equivalent embodiments, the first LP is configured to: abstain from transmitting the cardiac event message indicative of the cardiac event to the second LP, when there is not a determination that at least one of the plurality of predetermined conditions is satisfied; and transmit the cardiac event message indicative of the cardiac event to the second LP, when there is a determination that at least one of the plurality of predetermined conditions is satisfied.

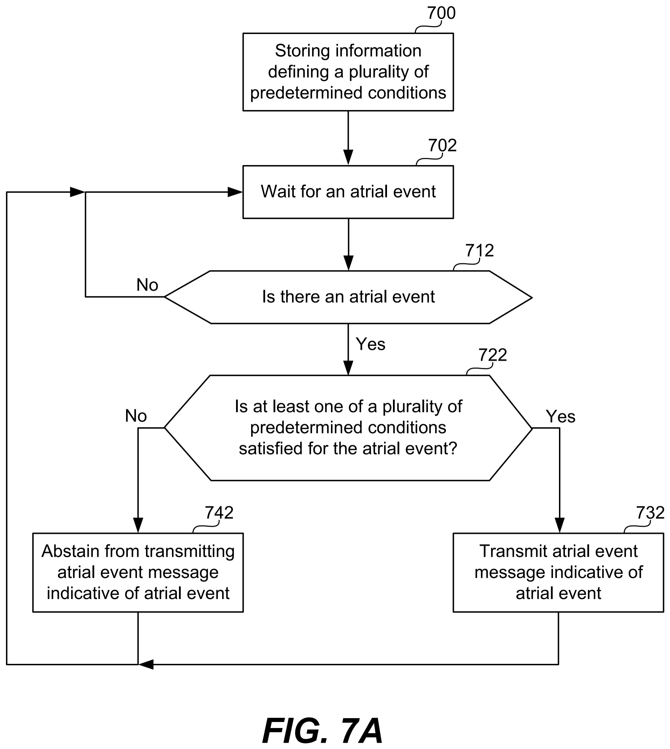

The high level flow diagram of FIG. 7A will now be used to summarize specific methods according to certain embodiments of the present technology that are for use with a multi-chamber LP system including at least an aLP (e.g., 102a) and a vLP (e.g., 102b), wherein the aLP is configured to be implanted in or on an atrial cardiac chamber (e.g., the right atrial cardiac chamber), and the vLP is configured to be implanted in or on a ventricular cardiac chamber (e.g., the right ventricular cardiac chamber). Referring to FIG. 7A, the steps performed therein are performed by the aLP that is configured to selectively communicate with the vLP of the multi-chamber LP system, which can be a dual-chamber LP system, but is not limited thereto. Step 700 involves the aLP storing information defining a plurality of predetermined conditions. The predetermined conditions can be specified by default by the manufacturer of the aLP, or can be programmable and thereby selectable by a user (e.g., a physician or clinician). Step 702 involves waiting for an atrial event associated with the atrial chamber (e.g., the right atrial chamber) in or on which the aLP is implanted. The atrial event that is waited for at step 702 can be an intrinsic atrial event that is sensed by the aLP, which can also be referred to as a sensed atrial event. The atrial event that is waited for at step 702 can alternatively be a paced atrial event that is caused by the aLP, or is about to be caused by the aLP. Such a paced atrial event is a type of paced event. For the following discussion, where an i2i message is sent from an aLP to a vLP, the i2i message can be referred to more specifically as an a2v i2i message, or more succinctly as an a2v message. Similarly, where an i2i message is sent from a vLP to an aLP, the i2i message can be referred to more specifically as a v2a i2i message, or more succinctly as a v2a message.

Still referring to FIG. 7A, step 712 involves determining whether there is an atrial event, which as noted above can be an intrinsic atrial event or a paced atrial event. Accordingly, the answer to the determination at step 712 will be Yes in response to the aLP sensing an intrinsic atrial event, i.e., in response to a sensed atrial event. Where the aLP is configured to deliver pacing stimulation to the atrium (in or on which the aLP is implanted) in response to an AA escape interval timer expiring without an intrinsic atrial event being detected (during an AA interval that is reset each time an atrial event is sensed or paced), then the answer to the determination at step 712 will be Yes in response to the AA interval timer expiring. For another example, where the aLP is configured to deliver pacing stimulation to the atrium (in or on which the aLP is implanted) in response to a VA interval timer expiring without an intrinsic atrial event being detected (during a VA interval that is reset when the aLP receives a ventricular event message from the vLP), then the answer to the determination at step 712 will be Yes in response to the VA interval timer expiring. More generally, the answer to the determination at step 712 can be Yes just prior to (e.g., approximately 1 to 5 msec before) the aLP delivering pacing stimulation to the atrium (in or on which the aLP is implanted), while the aLP is delivering the pacing stimulation to the atrium, or just after (e.g., approximately 1 to 5 msec after) the aLP delivered the pacing stimulation to the atrium. The paced atrial event is a type of paced event, as noted above.

When the answer to the determination at step 712 is No, flow returns to step 702 and the aLP continues to wait for an atrial event, which as noted above can be a sensed event or a paced event. When the answer to the determination at step 712 is Yes, either because an intrinsic atrial event is sensed, or a paced atrial event is caused (or about to be caused), then flow goes to step 722. More generally, flow can go from step 712 to step 722 in response to a sensed atrial event, a paced atrial event having been caused, or a paced atrial event about to be caused.

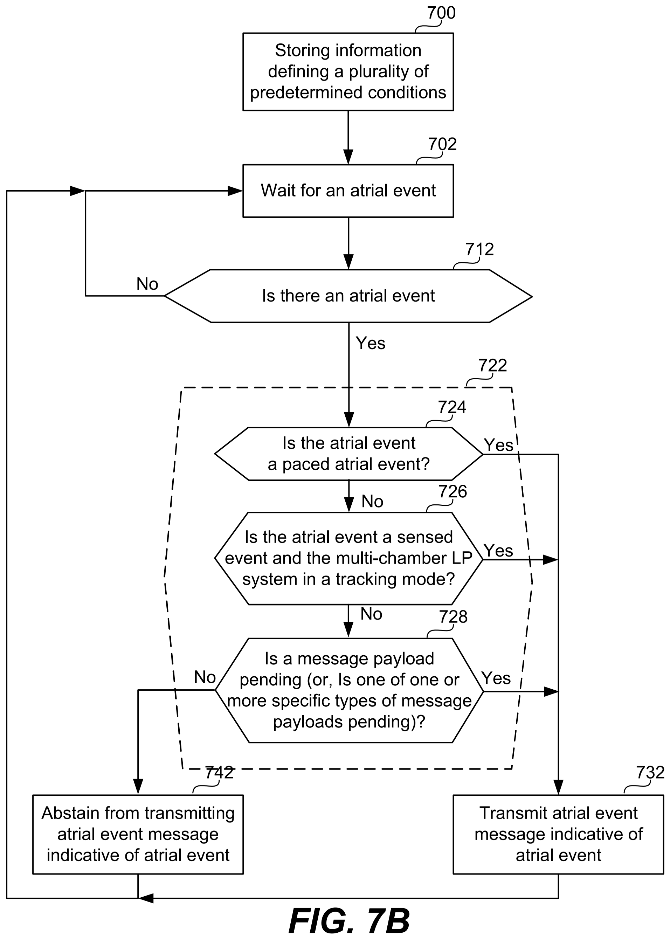

At step 722 there is a determination of whether at least one of the plurality of predetermined conditions is satisfied for the atrial event (which had caused the answer to the determination at step 712 to be Yes). Additional details of step 722, in accordance with specific embodiments of the present technology, are described below with reference to FIG. 7B.

Still referring to FIG. 7A, when the answer to the determination at step 722 is Yes, flow goes to step 732. Step 732 involves transmitting, from the aLP to the vLP, an atrial event message indicative of the atrial event (which had caused the answer to the determination at step 712 to be Yes). Following step 732, flow returns to step 702 at which the aLP waits for a next atrial event.

When the answer to the determination at step 722 is No, flow goes to step 742. Step 742 involves the aLP abstaining from transmitting to the vLP an atrial event message indicative of the atrial event (which had caused the answer to the determination at step 712 to be No). Following step 742, flow returns to step 702 at which the aLP waits for a next atrial event. The flow diagram of FIG. 7A could have equivalently shown flow returning from step 722 directly back to step 702 when the answer to the determination at step 722 is No, since in such a flow diagram the transmitting at step 732 would be skipped (and thus, abstained from being performed) whenever that answer to the determination at step 722 is No.

Embodiments described herein can be used by a multi-chamber LP system including an aLP (e.g., 102a) and a vLP (e.g., 102b) to conserve substantially more energy (and thereby further increase device longevity) than would be the case if only a single type of predetermined condition being satisfied (e.g., the multi-chamber LP system being in a non-tracking mode) caused the aLP to abstain from transmitting an atrial event message to the vLP of the system. Further, by not just using a single type of predetermined condition as the deciding factor for determining whether or not an atrial event message is to be transmitted from the aLP to the vLP, there can be situations where an atrial event message is transmitted from the aLP to the vLP when the system is in a non-tracking mode because it is beneficial to the operation of the multi-chamber LP system. In other words, for reasons other than enabling the vLP to track the aLP it may be beneficial for the aLP to still send a cardiac event message to the vLP when the system is in a non-tracking mode, e.g., to allow for blanking by the vLP, and/or to enable the aLP to transfer a useful payload message to the vLP as soon as possible.

Additional details of step 722, in accordance with specific embodiments of the present technology, will now be described with reference to FIG. 7B. Referring to FIG. 7B, steps 702 and 712 are the same as steps 702 and 712 in FIG. 7A, and thus, need not be described again. In FIG. 7B, steps 724, 726, and 728 are sub-steps of step 722 introduced above with reference to FIG. 7A. As explained above, flow goes to step 722 when the answer to the determination at 712 is Yes. More specifically, when there is a sensed atrial event or a paced atrial event (which had caused the answer to the determination at step 712 to be Yes), flow goes to step 724.

At step 724 there is a determination of whether the atrial event (which had caused the answer to the determination at step 712 to be Yes) is a paced atrial event. If the answer to the determination at step 724 is Yes, then flow goes to step 732, at which the aLP transmits to the vLP an atrial event message indicative of the paced atrial event, which had caused the answer to the determination at step 724 to be Yes. Such an atrial event message indicative of the paced atrial event can also be referred to more succinctly as a paced atrial event message, which is a type of paced event message. It is beneficial for the aLP to transmit a paced atrial event message to the vLP because receipt thereof by the vLP can cause the vLP to initiate a post-atrial pacing blanking response that reduces the probability of the vLP falsely detecting an atrial paced event as a ventricular sensed event. More specifically, in certain embodiments, receipt of the paced atrial event message by the vLP causes the vLP to initiate a post atrial ventricular blanking (PAVB) interval during which the vLP blanks (ignores or disables) sense inputs of vLP to prevent the vLP from falsely detecting an atrial pacing pulse as a ventricular depolarization. If the answer to the determination at step 724 is No, then flow goes to step 726.

At step 726 there is a determination of whether the atrial event is a sensed atrial event and the multi-chamber LP system (e.g., a dual-chamber LP system) is in a tracking mode, as opposed to a non-tracking mode. If the answer to the determination is at step 726 is Yes, i.e., if the atrial event is a sensed arial event and the multi-chamber LP system is indeed in a tracking mode, then flow goes to step 732, at which the aLP transmits to the vLP an atrial event message. When the multi-chamber LP system is in a tracking mode, the vLP delivers pacing stimulation to a ventricular chamber (in or on which the vLP is implanted) at an AV delay following an atrial sensed or paced event, if the vLP does not detect an intrinsic ventricular event during an AV interval. Example tracking modes that an aLP and a vLP of a multi-chamber LP system can collectively provide include DDD, DDDR, VDD, VDDR, DDT, and DDTR operational modes. Accordingly, when the multi-chamber LP system is in a tracking mode, the vLP will attempt to pace the ventricular chamber (in or on which the vLP is implanted) at the same rate as the atrial rate and offset by an AV delay. Accordingly, it is beneficial for the aLP to transmit atrial event messages to the vLP when the multi-chamber LP system is in a tracking mode to maintain coordinated contractions of the atrial and ventricular chambers.

By contrast, when the multi-chamber LP is in a non-tracking mode, the vLP delivers pacing stimulation to a ventricular chamber (in or on which the vLP is implanted) at a rate that is independent of a sensed atrial rate. Example non-tracking modes that an aLP and a vLP of a multi-chamber LP system can collectively provide include DDI, DDIR, VDI, VDIR, DOO, and DOOR operational modes. The multi-chamber LP system may switch from a tracking mode to a non-tracking mode when the atrial rate exceeds a specified tracking rate threshold, which may occur when a patient is experiencing an episode of atrial flutter (AFL) or atrial fibrillation (AF). The switch from a tracking mode to a non-tracking mode beneficially prevents the vLP from pacing the ventricular chamber (in or on which the vLP is implanted) at a dangerously high rate. Because the vLP delivers pacing stimulation to the ventricular chamber (in or on which the vLP is implanted) at a rate that is independent of the sensed atrial rate, while the multi-chamber LP system is in a non-tracking mode, there may be little or no benefit of the vLP receiving atrial sensed event messages from the aLP on a beat-by-beat basis (i.e., every cardiac cycle) while the multi-chamber LP system is in a non-tracking mode. Accordingly, the aLP can conserve energy by not transmitting atrial sensed event messages to the vLP while the multi-chamber LP system is in a non-tracking mode, since those atrial sensed event messages are not used by the vLP to control the timing of the ventricular pacing performed by the vLP. If the answer to the determination at step 726 is No, then flow goes to step 728.

At step 728 there is a determination of whether a message payload is pending that is to be transmitted as part of the atrial event message indicative of the atrial event (which had caused the answer to the determination at step 712 to be Yes). If the answer to the determination at step 728 is Yes, i.e., if a message payload is indeed pending, then flow goes to step 732. At step 732 the aLP transmits to the vLP an atrial event message that includes the message payload that is pending. It is also noted that if a message payload is pending when an atrial event message is transmitted in response to a determination at step 724 or step 726 being Yes, then such a pending message payload will also be sent in those instances. For example, if the atrial event is a paced event and there is also a message payload pending, when the atrial event message indicative of the paced atrial event is transmitted from the aLP to the vLP (in response to the answer to the determination at step 724 being Yes) the message payload that is pending will also be sent as part of that atrial event message. For another example, if the multi-chamber LP system is in a tracking mode when there is a sensed (or paced) atrial event, if a message payload is pending at that time, when the atrial event message indicative of the sensed (or paced) atrial event is transmitted from the aLP to the vLP the message payload that is pending will also be sent as part of that atrial event message. A benefit of transmitting an atrial event message including a message payload when the message payload is pending is that information (e.g., one or more indicators and/or commands) included in the message payload can be used by the vLP to modify its operation as soon as possible. For example, if a pending message payload includes a pacing rate indicator, then transmitting the pacing rate indicator in the message payload of an atrial event message enables the vLP (that receives the atrial event message) to adjust its ventricular pacing rate responsive to the pacing rate indicator included in the message payload. Further examples of types of a pending message payload that may be beneficial to transmit from the aLP to the vLP as soon as possible, were described above in the discussion of step 628, and thus, need not be repeated.

The determinations that are made at the steps 724, 726, and 728 in FIG. 7B, which can be considered sub-steps of the step 722 introduced in the discussion of FIG. 7A, are collectively an example of the determination performed at step 722. In other words, sub-steps 724, 726, and 728 can be performed to determine whether at least one of a plurality of predetermined conditions is satisfied at step 722. Examples of the predetermined conditions (which when at least one of them is satisfied results in an atrial event message indicative of an atrial event being transmitted from the aLP to the vLP at an instance of step 732) include: an atrial event is a paced atrial event; the atrial event is a sensed atrial event and the multi-chamber LP system is in a tracking mode; and a message payload is pending. It is also within the scope of the embodiments described herein that there can be additional and/or alternative predetermined conditions, where if at least one of the predetermined conditions is found to be satisfied, results in an atrial event message indicative of an atrial event being transmitted from the aLP to the vLP. In other words, there can be additional and/or alternative determinations performed at step 722, or at sub-steps thereof.

Further, it is noted that the order of the steps 724, 726, and 728 can be rearranged, wherein as noted above such steps can be referred to as sub-steps of step 722. More specifically, the order of the steps can instead be steps 726, 728, 724, in that order. Alternatively, the order of the steps can instead be steps 728, 724, 726, in that order. In still another alternative, the order of the steps can be step 728, 726, 724, in that order. When the order of such steps is rearranged, flow will go to step 732 whenever the answer to any one of the determinations in such steps is Yes. Additionally, when the order of such steps is rearranged, flow should go step 742 when the answer to all of the determinations in such steps is No. In other words, whichever one of the steps 724, 726, and 728 is the last to be performed should include an arrow to step 742 (or equivalent back to step 702) responsive to the answer to the determination made at the last performed step being No.

Referring again to step 728, in certain embodiments an atrial event message will be sent from the aLP to the vLP if a message payload is pending, regardless of the type of message payload that is pending. In other words, in certain embodiments the answer to the determination at step 728 will be Yes, regardless of the type of message payload that is pending at the aLP.

In alternative embodiments, not all message payloads are treated the same. Rather, in such alternative embodiments only one or more specific types of message payload if pending at the aLP will result in the answer to the determination in a variation on step 728 being Yes. More specially, in the alternative embodiment, at step 728 there is a determination of whether a message payload, which is to be transmitted from the aLP to the vLP as part of the atrial event message indicative of the atrial event, is pending and is one of one or more predetermined types of message payload. The one or more predetermined types of message payload can be those that are deemed to be sufficiently important such that they should be provided from the aLP to the vLP as soon as possible, so that the vLP can modify its behavior accordingly as soon as possible. In such embodiments, for other types of message payload which are deemed to be less important, the operation of the multi-chamber LP system may be unaffected if the aLP does not provide the message payload to the aLP as soon as possible, e.g., if the message payload simply includes a housekeeping type of command, or some other type of command or indicator for which it is not important that the vLP modify its behavior accordingly as soon as possible. Examples of one or more predetermined types of message payload that can be deemed to be sufficient important such that they should be provided from the aLP to the vLP as soon as possible, and thus should result in the answer to step 728 being Yes, were discussed above with reference to step 628, and thus, need not be repeated.

As noted above, a reason it is beneficial for the aLP to transmit a paced atrial event message to the vLP is because receipt thereof by the vLP causes the vLP to initiate a PAVB interval during which the vLP blanks sense inputs of vLP to prevent the vLP from falsely detecting an atrial pacing pulse as a ventricular depolarization. However, in embodiments where the vLP is configured to detect crosstalk caused by the aLP delivering pacing stimulation, the vLP can initiate a PAVB (or equivalent) interval in response to the vLP detecting such crosstalk, instead of in response to the vLP receiving a paced atrial event message from the aLP. In other words, in embodiments where the vLP is configured to detect crosstalk caused by the aLP delivering pacing stimulation to the atrium in or on which the aLP is implanted, the vLP is not reliant on paced atrial event messages to initiate a PAVB interval, but rather, the vLP can instead initiate a PAVB interval in response to detecting the crosstalk caused by the aLP delivering the pacing stimulation. Accordingly, the vLP can rely on crosstalk detection to initiate a PAVB interval.

The vLP can be configured to detect crosstalk, for example, using one of the techniques described in the description of FIGS. 2-8 of U.S. Patent No. 10,182,765, titled "Systems and Methods for Classifying Signals of Interest in a Cardiac Rhythm Management Device". Additional details explaining how an LP can be configured to detect crosstalk caused by another LP delivering pacing stimulation were described above, and thus, need not be repeated. In an embodiment, where the vLP is configured to detect crosstalk caused by the aLP delivering pacing stimulation to the atrial chamber (in or on which the aLP is implanted), and the vLP is configured to start a blanking interval in response to detecting such crosstalk, step 724 can be removed from the flow diagram of FIG. 7B. In such an embodiment, when the answer to the determination at step 712 is Yes, flow can go directly from step 712 to step 726. Alternatively, when the answer to the determination at step 712 is Yes, flow can go directly from step 712 to step 728, and the order of steps 726 and 728 can be rearranged such that step 728 is performed prior to step 726.

In embodiments summarized above with reference to FIGS. 7A and 7B, for each atrial event associated with the atrial chamber in or on which aLP is implanted, of a plurality of atrial events, the aLP is configured to: abstain from transmitting the atrial event message indicative of the atrial event to the vLP, when there is a determination that none of the plurality of predetermined conditions is satisfied; and transmit the atrial event message indicative of the atrial event to the vLP, when there is a determination that at least one of the plurality of predetermined conditions is satisfied. In alternative yet equivalent embodiments, the aLP is configured to: abstain from transmitting the atrial event message indicative of the atrial event to the vLP, when there is not a determination that at least one of the plurality of predetermined conditions is satisfied; and transmit atrial cardiac event message indicative of the atrial event to the vLP, when there is a determination that at least one of the plurality of predetermined conditions is satisfied. Unless specifically stated otherwise, in the embodiments described above with reference to FIGS. 6A, 6B, 7A, and 7B, each of the cardiac events associated with a cardiac chamber in (or on) which an LP is implanted (for which the LP determines whether or not a corresponding cardiac event message should be sent to another LP) is a cardiac event this is nonconcurrent with the blanking and refractory periods of the LP.

Embodiments of the present technology can additionally, or alternatively, be used to determine whether messages for other types of events (in additional to, or instead of cardiac events) should be transmitted from a first LP to a second LP, e.g., from an aLP to a vLP, or vice versa. An example of such an event is an event where an interval timer of a first LP expires without an intrinsic event of the first cardiac chamber (in or on which the first LP is implanted) being detected by the first LP, and the first LP does not deliver a pacing pulse to the cardiac chamber in or on which the first LP is implanted. In such an event, there is not a "cardiac event" sensed or paced by the first LP, but the first LP may nevertheless want to inform the second LP of the event. In accordance with certain embodiments, the first LP can determine for such an alternative type of event (and/or one or more other types of events), whether at least one of a plurality of predetermined conditions is satisfied, and the first LP can abstain from transmitting an event message indicative of the event to the second LP, or transmit the event message indicative of the event to the second LP, based on whether there is a determination that at least one of the plurality of predetermined conditions is satisfied. Referring back to FIG. 6A, in such an alternative embodiment, step 602 involves waiting for an event (which may be something other than a cardiac event, but optionally also can be a cardiac event), and step 612 involves determining whether there is an event associated with the chamber in or on which the first LP is implanted. Similarly, the message that is transmitted at step 632, or not transmitted at step 642, may be a type of event message other than a cardiac event message. Similarly, in each of the steps shown in and described above with reference to the flow diagram in FIG. 6B, each reference to a cardiac event can instead be a reference to another type of event for which a first LP may send an event message to second LP, to thereby inform the second LP of the event. Similarly, in each of the steps shown in and described above with reference to the flow diagrams in FIGS. 7A and 7B, each reference to an atrial event can instead be a reference to another type of event for which an aLP may send an event message to a vLP, to thereby inform the vLP of the event.

An aspect of the present technology relates to a multi-chamber implantable LP system (e.g., 100), comprising a first LP (e.g., 102a, 102b) configured to be implanted in or on a first cardiac chamber, and a second LP (e.g., 102a, 102b) configured to be implanted in or on a second cardiac chamber. For each cardiac event that is nonconcurrent with blanking and refractory periods of the first LP, of a plurality of cardiac events associated with the first cardiac chamber that are nonconcurrent with blanking and refractory periods of the first LP, the first LP is configured to determine for the cardiac event whether at least one of a plurality of predetermined conditions is satisfied. Additionally, for each cardiac event, of the plurality of cardiac events associated with the first cardiac chamber, the first LP is configured to abstain from transmitting a cardiac event message indicative of the cardiac event to the second LP, or transmit the cardiac event message indicative of the cardiac event to the second LP, based on whether there is a determination that at least one of the plurality of predetermined conditions is satisfied.

In an embodiment, for each cardiac event associated with the first cardiac chamber that is nonconcurrent with blanking and refractory periods of the first LP, of the plurality of cardiac events that are nonconcurrent with blanking and refractory periods of the first LP, the first LP is configured to: abstain from transmitting the cardiac event message indicative of the cardiac event to the second LP, when there is a determination that none of the plurality of predetermined conditions is satisfied; and transmit the cardiac event message indicative of the cardiac event to the second LP, when there is a determination that at least one of the plurality of predetermined conditions is satisfied.

In an embodiment, one of the plurality of predetermined conditions is satisfied when the cardiac event comprises a paced cardiac event.

In an embodiment, the system is configured to operate in one of a tracking mode or a non-tracking mode at a time, and to switch from the tracking mode or the non-tracking mode to the other, and vice versa; and one of the plurality of predetermined conditions is satisfied when the system is operating in the tracking mode and the cardiac event comprises a sensed cardiac event.

In an embodiment, one of the plurality of predetermined conditions is satisfied when a message payload is pending at the first LP, or when a message payload is pending at the first LP that is one of one or more predetermined types of message payload.

In an embodiment, the system is configured to operate in one of a tracking mode or a non-tracking mode at a time, and to switch from the tracking mode or the non-tracking mode to the other, and vice versa; a first one of the plurality of predetermined conditions is satisfied when the cardiac event comprises a paced cardiac event; a second one of the plurality of predetermined conditions is satisfied when the system is operating in the tracking mode and the cardiac event comprises a sensed cardiac event; and a third one of the plurality of predetermined conditions is satisfied when a message payload is pending at the first LP, or when a message payload is pending at the first LP that is one of one or more predetermined types of message payload.

In an embodiment, the system is configured to operate in one of a tracking mode or a non-tracking mode at a time, and to switch from the tracking mode or the non-tracking mode to the other, and vice versa; and a first one of the plurality of predetermined conditions is satisfied when the system is operating in the tracking mode and the cardiac event comprises a sensed cardiac event; and a second one of the plurality of predetermined conditions is satisfied when a message payload is pending at the first LP, or when a message payload is pending at the first LP that is one of one or more predetermined types of message payload.

In an embodiment, for each cardiac event associated with the first cardiac chamber that is nonconcurrent with blanking and refractory periods of the first LP, of the plurality of cardiac events that are nonconcurrent with blanking and refractory periods of the first LP, the first LP is configured to: abstain from transmitting the cardiac event message indicative of the cardiac event to the second LP, when there is not a determination that at least one of the plurality of predetermined conditions is satisfied; and transmit the cardiac event message indicative of the cardiac event to the second LP, when there is a determination that at least one of the plurality of predetermined conditions is satisfied.

In an embodiment, the first LP comprises an aLP configured to be implanted in or on an atrial chamber; and the second LP comprises a vLP configured to be implanted in or on a ventricular chamber. In a specific such embodiment, for each atrial event that is nonconcurrent with blanking and refractory periods of the aLP, of a plurality of atrial events associated with the atrial chamber that are nonconcurrent with blanking and refractory periods of the aLP, the aLP is configured to determine for the atrial event whether at least one of a plurality of predetermined conditions is satisfied; and abstain from transmitting an atrial event message indicative of the atrial event to the vLP, or transmit the atrial event message indicative of the atrial event to the vLP, based on whether there is a determination that at least one of the plurality of predetermined conditions is satisfied.

In an embodiment, the first LP comprises a vLP configured to be implanted in or on a ventricular chamber; and the second LP comprises an aLP configured to be implanted in or on an atrial chamber. In a specific such embodiment, for each ventricular event that is nonconcurrent with blanking and refractory periods of the vLP, of a plurality of ventricular events associated with the ventricular chamber that are nonconcurrent with blanking and refractory periods of the vLP, the vLP is configured to determine for the ventricular event whether at least one of a plurality of predetermined conditions is satisfied; and abstain from transmitting a ventricular event message indicative of the ventricular event to the aLP, or transmit the ventricular event message indicative of the ventricular event to the aLP, based on whether there is a determination that at least one of the plurality of predetermined conditions is satisfied.

In an embodiment, each LP, of the first LP and the second LP, comprising a respective controller (e.g., 112), one or more respective sense circuits (e.g., 132), one or more respective pulse generators (e.g., 116), a respective pair of electrodes (e.g., 108a, 108b), and a respective battery (e.g., 114) that powers the respective controller, the respective one or more sense circuits, and the respective one or more pulse generators of the LP. The respective one or more pulse generators and the respective pair of electrodes, of each LP, are configured to output pacing pulses and output conducted communication pulses. The respective one or more sense circuits and the respective pair of electrodes, of each LP, are configured to sense an EGM and sense conducted communication pulses. In such an embodiment, the first LP abstaining from transmitting a said cardiac event message to the second LP reduces an amount of power consumed from the battery of the first LP compared to when the first LP transmits a said cardiac event message to the second LP.

In an embodiment, the first LP is configured to disable sensing of events or to ignore sensed events that are concurrent with blanking periods of the first LP, and the first LP is configured to disable sensing of events or to ignore sensed events that are concurrent with refractory periods of the first LP. Such sensing can be disabled, e.g., by disabling a sense circuit (e.g., 132) of the LP. Such sensing can be ignored, e.g., by ignoring the output of the sense circuit (e.g., 132) when the sense circuit is enabled.

A further aspect of the present technology relates to a method for use by a first LP (e.g., 102a, 102b) of a multi-chamber implantable LP system (e.g., 100) comprising the first LP (e.g., 102a, 102b) configured to be implanted in or on a first cardiac chamber and a second LP (e.g., 102a, 102b) configured to be implanted in or on a second cardiac chamber, a method for use by the first LP. The method comprises the first LP determining, for each cardiac event that is nonconcurrent with blanking and refractory periods of the first LP of a plurality of cardiac events associated with the first cardiac chamber that are nonconcurrent with blanking and refractory periods of the first LP, whether at least one of a plurality of predetermined conditions is satisfied. The method also comprises the first LP selectively abstaining from transmitting a cardiac event message indicative of the cardiac event to the second LP, or transmitting the cardiac event message indicative of the cardiac event to the second LP, based on whether there is a determination for the cardiac event that at least one of the plurality of predetermined conditions is satisfied.

In an embodiment, the method includes the first LP abstaining from transmitting a said cardiac event message indicative of a first said cardiac event to the second LP, in response to the first LP determining for the first cardiac event that none of the plurality of predetermined conditions is satisfied. The method also includes the first LP transmitting a said cardiac event message indicative of a second cardiac event to the second LP, in response to the first LP determining for the second cardiac event that at least one of the plurality of predetermined conditions is satisfied.

In an embodiment, one of the plurality of predetermined conditions is satisfied when the cardiac event comprises a paced cardiac event.

In an embodiment, the multi-chamber implantable LP system is configured to operate in one of a tracking mode or a non-tracking mode at a time, and to switch from the tracking mode or the non-tracking mode to the other, and vice versa; and one of the plurality of predetermined conditions is satisfied when the multi-chamber implantable LP system is operating in the tracking mode and the cardiac event comprises a sensed cardiac event.

In an embodiment, one of the plurality of predetermined conditions is satisfied when a message payload is pending at the first LP, or when a message payload is pending at the first LP that is one of one or more predetermined types of message payload.

In an embodiment, the multi-chamber implantable LP system is configured to operate in one of a tracking mode or a non-tracking mode at a time, and to switch from the tracking mode or the non-tracking mode to the other, and vice versa; a first one of the plurality of predetermined conditions is satisfied when the cardiac event comprises a paced cardiac event; a second one of the plurality of predetermined conditions is satisfied when the multi-chamber implantable LP system is operating in the tracking mode and the cardiac event comprises a sensed cardiac event; and a third one of the plurality of predetermined conditions is satisfied when a message payload is pending at the first LP, or when a message payload is pending at the first LP that is one of one or more predetermined types of message payload.

In an embodiment, the multi-chamber implantable LP system is configured to operate in one of a tracking mode or a non-tracking mode at a time, and to switch from the tracking mode or the non-tracking mode to the other, and vice versa; a first one of the plurality of predetermined conditions is satisfied when the multi-chamber implantable LP system is operating in the tracking mode and the cardiac event comprises a sensed cardiac event; and a second one of the plurality of predetermined conditions is satisfied when a message payload is pending at the first LP, or when a message payload is pending at the first LP that is one of one or more predetermined types of message payload.

In an embodiment, the method includes the first LP abstaining from transmitting a said cardiac event message indicative of a first said cardiac event to the second LP, in response to the first LP not determining for the first cardiac event that at least one of the plurality of predetermined conditions is satisfied. In such an embodiment, the method also includes the first LP transmitting a said cardiac event message indicative of a second cardiac event to the second LP, in response to the first LP determining for the second cardiac event that at least one of the plurality of predetermined conditions is satisfied.

In an embodiment, the first LP comprises an aLP (e.g., 102a) configured to be implanted in or on an atrial chamber; and a second LP comprises a vLP (e.g., 102b) configured to be implanted in or on a ventricular chamber. In a specific such embodiment, for each atrial event that is nonconcurrent with blanking and refractory periods of the aLP, of a plurality of atrial events associated with the atrial chamber that are nonconcurrent with blanking and refractory periods of the aLP, the method comprises the aLP determining, for the atrial event, whether at least one of a plurality of predetermined conditions is satisfied; and the aLP abstaining from transmitting an atrial event message indicative of the atrial event to the vLP, or transmitting the atrial event message indicative of the atrial event to the vLP, based on whether there is a determination that at least one of the plurality of predetermined conditions is satisfied.

In an embodiment, the first LP comprises a vLP (e.g., 102b) configured to be implanted in or on a ventricular chamber; the second LP comprises an aLP (e.g., 102a) configured to be implanted in or on an atrial chamber. In a specific such embodiment, for each ventricular event that is nonconcurrent with blanking and refractory periods of the vLP, of a plurality of ventricular events associated with the ventricular chamber that are nonconcurrent with blanking and refractory periods of the vLP, the method comprises: the vLP determining, for the ventricular event, whether at least one of a plurality of predetermined conditions is satisfied; and the vLP abstaining from transmitting a ventricular event message indicative of the ventricular event to the aLP, or transmitting the ventricular event message indicative of the ventricular event to the aLP, based on whether there is a determination that at least one of the plurality of predetermined conditions is satisfied.

In an embodiment, each LP, of the first LP and the second LP, comprises a respective controller (e.g., 112), one or more respective sense circuits (e.g., 132), one or more respective pulse generators (e.g., 116), a respective pair of electrodes (e.g., 108a, 108b), and a respective battery (e.g., 114) that powers the respective controller, the respective one or more sense circuits, and the respective one or more pulse generators of the LP. The respective one or more pulse generators and the respective pair of electrodes, of each LP, are configured to output pacing pulses and output conducted communication pulses. The respective one or more sense circuits and the respective pair of electrodes, of each LP, are configured to sense an EGM and sense conducted communication pulses.

In an embodiment, the first LP abstains from transmitting a said cardiac event message to the second LP in order to reduce an amount of power consumed from the battery of the first LP compared to when the first LP transmits a said cardiac event message to the second LP.

In an embodiment, the method includes the first LP disabling sensing of events or ignoring sensed events that are concurrent with blanking periods of the first LP, and the first LP is disabling sensing of events or ignoring sensed events that are concurrent with refractory periods of the first LP

The various embodiments discussed in the foregoing in connection with FIGS. 1 to 7B also apply to these aspects of the invention.

Embodiments of the present technology have been described above with the aid of functional building blocks illustrating the performance of specified functions and relationships thereof. The boundaries of these functional building blocks have often been defined herein for the convenience of the description. Alternate boundaries can be defined so long as the specified functions and relationships thereof are appropriately performed. Any such alternate boundaries are thus within the scope of the claimed invention. For example, it would be possible to combine or separate some of the steps shown in FIGS. 6A, 6B, 7A, and 7B. It would also be possible to reorder some of the steps shown in FIG. 6B and 7B, as explained above. For another example, it is possible to change the boundaries of some of the blocks shown in FIG. 2. It is to be understood that the subject matter described herein is not limited in its application to the details of construction and the arrangement of components set forth in the description herein or illustrated in the drawings hereof. The subject matter described herein is capable of other embodiments and of being practiced or of being carried out in various ways. Also, it is to be understood that the phraseology and terminology used herein is for the purpose of description and should not be regarded as limiting. The use of "including," "comprising," or "having" and variations thereof herein is meant to encompass the items listed thereafter and equivalents thereof as well as additional items. Further, it is noted that the term "based on" as used herein, unless stated otherwise, should be interpreted as meaning based at least in part on, meaning there can be one or more additional factors upon which a decision or the like is made. For example, if a decision is based on the results of a comparison, that decision can also be based on one or more other factors in addition to being based on results of the comparison.

It is to be understood that the above description is intended to be illustrative, and not restrictive. For example, the above-described embodiments (and/or aspects thereof) may be used in combination with each other. In addition, many modifications may be made to adapt a particular situation or material to the teachings of the embodiments of the present technology without departing from its scope. While the dimensions, types of materials and coatings described herein are intended to define the parameters of the embodiments of the present technology, they are by no means limiting and are exemplary embodiments. Many other embodiments will be apparent to those of skill in the art upon reviewing the above description. The scope of the embodiments of the present technology should, therefore, be determined with reference to the appended claims, along with the full scope of equivalents to which such claims are entitled. In the appended claims, the terms "including" and "in which" are used as the plain-English equivalents of the respective terms "comprising" and "wherein." Moreover, in the following claims, the terms "first," "second," and "third," etc. are used merely as labels, and are not intended to impose numerical requirements on their objects.

## Claims

1. A multi-chamber implantable leadless pacemaker, LP, system (100), comprising:
a first LP (102a, 102b) configured to be implanted in or on a first cardiac chamber and configured to store information defining a plurality of predetermined conditions; and
a second LP configured (102b, 102a) to be implanted in or on a second cardiac chamber;
wherein for each cardiac event that is nonconcurrent with blanking and refractory periods of the first LP (102a, 102b), of a plurality of cardiac events associated with the first cardiac chamber that are nonconcurrent with the blanking and refractory periods of the first LP (102a, 102b), the first LP (102a, 102b) is configured to:
determine for the cardiac event whether at least one of the plurality of predetermined conditions is satisfied; and
abstain from transmitting a cardiac event message indicative of the cardiac event to the second LP (102b, 102a), or transmit the cardiac event message indicative of the cardiac event to the second LP (102b, 102a), based on whether there is a determination that at least one of the plurality of predetermined conditions is satisfied.

2. The multi-chamber implantable LP system (100) of claim 1, wherein for each cardiac event associated with the first cardiac chamber that is nonconcurrent with the blanking and refractory periods of the first LP (102a, 102b), of the plurality of cardiac events that are nonconcurrent with the blanking and refractory periods of the first LP (102a, 102b), the first LP (102a, 102b) is configured to:
abstain from transmitting the cardiac event message indicative of the cardiac event to the second LP (102b, 102a), when there is a determination that none of the plurality of predetermined conditions is satisfied; and
transmit the cardiac event message indicative of the cardiac event to the second LP (102b, 102a), when there is a determination that at least one of the plurality of predetermined conditions is satisfied.

3. The multi-chamber implantable LP system (100) of claim 2, wherein one of the plurality of predetermined conditions is satisfied when the cardiac event that is nonconcurrent with the blanking and refractory periods of the first LP (102a, 102b) comprises a paced cardiac event.

4. The multi-chamber implantable LP system (100) of any one of claims 2 or 3, wherein
the system (100) is configured to operate in one of a tracking mode or a non-tracking mode at a time, and to switch from the tracking mode or the non-tracking mode to the other, and vice versa; and
one of the plurality of predetermined conditions is satisfied when the system (100) is operating in the tracking mode and the cardiac event that is nonconcurrent with the blanking and refractory periods of the first LP (102a, 102b) comprises a sensed cardiac event.

5. The multi-chamber implantable LP system (100) of any one of claims 2 through 4, wherein one of the plurality of predetermined conditions is satisfied when a message payload is pending at the first LP (102a, 102b), or when a message payload is pending at the first LP (102a, 102b) that is one of one or more predetermined types of message payload.

6. The multi-chamber implantable LP system (100) any one of claims 2 through 5, wherein
the system (100) is configured to operate in one of a tracking mode or a non-tracking mode at a time, and to switch from the tracking mode or the non-tracking mode to the other, and vice versa;
a first one of the plurality of predetermined conditions is satisfied when the system (100) is operating in the tracking mode and the cardiac event that is nonconcurrent with the blanking and refractory periods of the first LP (102a, 102b) comprises a sensed cardiac event; and
a second one of the plurality of predetermined conditions is satisfied when the cardiac event that is nonconcurrent with the blanking and refractory periods of the first LP (102a, 102b) comprises a paced cardiac event.

7. The multi-chamber implantable LP system (100) of claim 6, wherein a third one of the plurality of predetermined conditions is satisfied when a message payload is pending at the first LP (102a, 102b), or when a message payload is pending at the first LP (102a, 102b) that is one of one or more predetermined types of message payload.

8. The multi-chamber implantable LP system (100) of any one of claims 1 through 7, wherein
the first LP (102a, 102b) comprises an atrial LP, aLP, (102a) configured to be implanted in or on an atrial chamber; and
the second LP (102b, 102a) comprises a ventricular LP, vLP, (102b) configured to be implanted in or on a ventricular chamber;
wherein for each atrial event that is nonconcurrent with the blanking and refractory periods of the aLP (102a), of a plurality of atrial events associated with the atrial chamber that are nonconcurrent with the blanking and refractory periods of the aLP (102a), the aLP (102a) is configured to:
determine for the atrial event whether at least one of a plurality of predetermined conditions is satisfied; and
abstain from transmitting an atrial event message indicative of the atrial event to the vLP (102b), or transmit the atrial event message indicative of the atrial event to the vLP (102b), based on whether there is a determination that at least one of the plurality of predetermined conditions is satisfied.

9. The multi-chamber implantable LP system (100) of any one of claims 1 through 7, wherein
the first LP (102a, 102b) comprises a ventricular LP, vLP, (102b) configured to be implanted in or on a ventricular chamber; and
the second LP (102b, 102a) comprises an atrial LP, aLP, (102a) configured to be implanted in or on an atrial chamber; and
wherein for each ventricular event that is nonconcurrent with the blanking and refractory periods of the vLP (102b), of a plurality of ventricular events associated with the ventricular chamber that are nonconcurrent with the blanking and refractory periods of the vLP (102b), the vLP (102b) is configured to:
determine for the ventricular event whether at least one of a plurality of predetermined conditions is satisfied; and
abstain from transmitting a ventricular event message indicative of the ventricular event to the aLP (102a), or transmit the ventricular event message indicative of the ventricular event to the aLP (102a), based on whether there is a determination that at least one of the plurality of predetermined conditions is satisfied.

10. The multi-chamber implantable LP system (100) of any one of claims 1 through 9, wherein
each LP (102a, 102b), of the first LP (102a, 102b) and the second LP (102b, 102a), comprising a respective controller (112), one or more respective sense circuits (132), one or more respective pulse generators (116), a respective pair of electrodes (108a, 108b), and a respective battery (114) that powers the respective controller (112), the respective one or more sense circuits (132), and the respective one or more pulse generators (116) of the LP (102a, 102b);
the respective one or more pulse generators (116) and the respective pair of electrodes (108a, 108b), of each LP (102a, 102b), are configured to output pacing pulses and output conducted communication pulses;
the respective one or more sense circuits (132) and the respective pair of electrodes (108a, 108b), of each LP (102a, 102b), are configured to sense an electrogram (EGM) and sense conducted communication pulses; and
the first LP (102a, 102b) abstaining from transmitting a said cardiac event message to the second LP (102b, 102a) reduces an amount of power consumed from the battery (114) of the first LP (102a, 102b) compared to when the first LP (102a, 102b) transmits a said cardiac event message to the second LP (102b, 102a).

11. A method for reducing communication burden in a multi-chamber implantable leadless pacemaker, LP, system (100) comprising a first LP (102a, 102b) configured to be implanted in or on a first cardiac chamber and a second LP (102b, 102a) configured to be implanted in or on a second cardiac chamber, the method comprising:
the first LP (102a, 102b) storing information defining a plurality of predetermined conditions;
the first LP (102a, 102b) determining, for each cardiac event that is nonconcurrent with the blanking and refractory periods of the first LP (102a, 102b) of a plurality of cardiac events associated with the first cardiac chamber that are nonconcurrent with the blanking and refractory periods of the first LP (102a, 102b), whether at least one of the plurality of predetermined conditions is satisfied; and
the first LP (102a, 102b) selectively abstaining from transmitting a cardiac event message indicative of the cardiac event that is nonconcurrent with the blanking and refractory periods of the first LP (102a, 102b) to the second LP (102b, 102a), or transmitting the cardiac event message indicative of the cardiac event that is nonconcurrent with the blanking and refractory periods of the first LP (102a, 102b) to the second LP (102b, 102a), based on whether there is a determination for the cardiac event that at least one of the plurality of predetermined conditions is satisfied.

12. The method of claim 11, wherein the method comprises:
the first LP (102a, 102b) abstaining from transmitting a said cardiac event message indicative of a first said cardiac event that is nonconcurrent with the blanking and refractory periods of the first LP (102a, 102b) to the second LP (102b, 102a), in response to the first LP (102a, 102b) determining for the first cardiac event that none of the plurality of predetermined conditions is satisfied; and
the first LP (102a, 102b) transmitting a said cardiac event message indicative of a second cardiac event that is nonconcurrent with the blanking and refractory periods of the first LP (102a, 102b) to the second LP (102b, 102a), in response to the first LP (102a, 102b) determining for the second cardiac event that at least one of the plurality of predetermined conditions is satisfied.

13. The method of claim 12, wherein one of the plurality of predetermined conditions is satisfied when the cardiac event comprises a paced cardiac event.

14. The method of any one of claims 12 or 13, wherein
the multi-chamber implantable LP system (100) is configured to operate in one of a tracking mode or a non-tracking mode at a time, and to switch from the tracking mode or the non-tracking mode to the other, and vice versa; and
one of the plurality of predetermined conditions is satisfied when the multi-chamber implantable LP system (100) is operating in the tracking mode and the cardiac event comprises a sensed cardiac event.

15. The method of any one of claims 12 through 14, wherein one of the plurality of predetermined conditions is satisfied when a message payload is pending at the first LP (102a, 102b), or when a message payload is pending at the first LP (102a, 102b) that is one of one or more predetermined types of message payload.
